# EUROPEAN PATENT APPLICATION

(11) **EP 4 209 589 A1**
(43) Date of publication of application: **12.07.2023**
(21) Application number: 21864464.9
(22) Date of filing: 06.09.2021
(51) Int. Cl.: C12N 15/09, C12N 15/63, C12N 15/864, C12N 9/78

(54) **MINIATURIZED CYTIDINE DEAMINASE-CONTAINING COMPLEX FOR MODIFYING DOUBLE-STRANDED DNA**

(30) Priority: 04.09.2020 JP 2020149419
(71) Applicant: National University Corporation Kobe University, Kobe-shi, Hyogo 657-8501 (JP)
(72) Inventor: NISHIDA, Keiji, Kobe-shi, Hyogo 657-8501 (JP); LI, Ang, Kobe-shi, Hyogo 657-8501 (JP); MITSUNOBU, Hitoshi, Kobe-shi, Hyogo 657-8501 (JP)
(74) Representative: Heubeck, Christian
(86) International application number: PCT/JP2021/032689
(87) International publication number: WO 2022/050413

(57) **Abstract**

The present disclosure provides a miniaturized cytidine deaminase-containing complex for modifying DNA, the complex being capable of miniaturizing cytidine deaminase and suppressing off-target effects while suppressing a reduction in the modification efficiency of a target site. The complex is formed by combining a nucleic acid sequence recognition module and cytidine deaminase, wherein: the nucleic acid sequence recognition module specifically binds to a target nucleotide sequence of double-stranded DNA; the cytidine deaminase is composed of (1) an amino acid sequence composed of a region of amino acid residues at the 30th position to 150th position of an amino acid sequence represented by SEQ ID NO: 1, (2) an amino acid sequence, which is an ortholog of a protein composed of an amino acid sequence represented by SEQ ID No:1, the amino acid sequence being composed of a region corresponding to the region in (1), (3) an amino acid sequence having one or several amino acids in the amino acid sequence in (1) or (2)deleted, substituted, inserted, and/or added, or (4) an amino acid sequence having at least 90% similarity to or being identical to the amino acid sequence in (1) or (2); and the targeted site of the double-stranded DNA is modified.

## Description

### [Technical Field]

The present disclosure relates to a complex for altering a double stranded DNA, which enables alteration of a targeted site of the double stranded DNA of a cell without a DNA double strand break, and a method of altering a double stranded DNA using said complex.

### [Background Art]

In recent years, genome editing has drawn attention as a technology for altering a gene/genomic region of interest in various organism species. For example, a method of recombination in a targeted locus in a DNA in a host plant cell or insect cell by using a zinc finger nuclease (ZFN), which is prepared from linking a zinc finger DNA binding domain and a non-specific DNA cleavage domain (Patent Literature 1); and a method of cleaving/modifying a targeted gene at a site within or adjacent to a specific nucleotide sequence by using a TALEN prepared by linking a transcription activator-like (TAL) effector, which is a DNA binding module of plant pathogenic *Xanthomonas spp.,* with a DNA endonuclease (Patent Literature 2) have been reported. Alternatively, *Streptococcus pyogenes* derived Cas9 nuclease is broadly used as a potent genome editing tool in eukaryotes having a repair pathway for DNA double strand breaks (DSB) (e.g., Patent Literature 3, Non Patent Literatures 1 and 2).

Target base editing mediated by a cytidine deaminase, which directly edits a nucleotide in a target locus without using a donor DNA comprising a homology arm for a target region, has also been demonstrated (e.g., Non Patent Literature 3). In view of utilizing DNA deamination instead of DNA cleavage mediated by a nuclease, this technology has low toxicity to cells and is capable of pinpoint introduction of a mutation. For this reason, not only utilization as a biomolecular tool for preparing genetically altered animals, but also for applications in medicine such as gene therapy is expected.

However, genome editing using a cytidine deaminase requires a cytidine deaminase for applications in medicine, such that the molecular weight of a complex used in genome editing increases, which is one of the obstructive factors for efficient delivery. In this regard, an attempt has been made to reduce the molecular weight of a cytidine deaminase by deleting a part of a region of the cytidine deaminase (Non Patent Literature 4).

### [Citation List]

### [Patent Literature]

[PTL 1] International Publication No. WO 2003/087341
[PTL 2] International Publication No. WO 2011/072246
[PTL 3] International Publication No. WO 2013/176772

### [Non Patent Literature]

[NPL 1] Mali, P. et al., Science 339 (6121): 823-826 (2013)
[NPL 2] Cong, L. et al., Science 339 (6121): 819-823 (2013)
[NPL 3] Nishida, K. et al., Science 353 (6305) : aaf8729 (2016)
[NPL 4] Tan J. et al., Nat Commun. 10(1): 439 (2019)

### [Summary of Invention]

### [Technical Problem]

However, it is reported that as the portions that are deleted from a wild-type cytidine deaminase increases, the efficiency of altering a target site of the complex of a compact cytidine deaminase and nickase Cas9 disclosed in Non Patent Literature 4 decreases. The complex further comprises a uracil-DNA glycosylase inhibitor (UGI). However, a UGI inhibits the function of uracil-DNA glycosylase which is important in repairing DNA. Thus, an undesirable off-target effect is expected to be enhanced. Therefore, the present disclosure provides a complex for altering a double stranded DNA, which comprises a compact cytidine deaminase, which has a reduced size while also suppressing a decrease in the efficiency of altering a target site, and is capable of achieving suppressing an off-target effect.

### [Solution to Problem]

The inventors have prepared a complex by removing a UGI from the complex disclosed in Non Patent Literature 4 described above. The inventors also prepared complexes by further deleting the N-terminus side region of the complex and investigated the efficiency of altering a target site with these complexes. As a result, it was unexpectedly found that the efficiency of alteration when a UGI was not used was 2/3 or less, even for a complex using CDA1 consisting of a region of positions 1 to 161 (i.e., CDA with a deletion of 32 amino acid regions from the C-terminus side of wild-type CDA; also referred to as CDA1Δ161), which was found to have high efficiency of alteration in Non Patent Literature 4 compared to a complex using wild-type CDA1, and the efficiency of alteration was 1/10, or less, compared to conventional Target-AID which is fused to the C-terminus via a linker. Furthermore, it was shown by these findings that alteration is significantly reduced in CDA1 with a deletion of two or more amino acid residues on the N-terminal side of CDA1Δ161. In view of these findings, the inventors reached the conclusion that achievement of high efficiency of alteration of a target site by the complex disclosed in Non Patent Literature 4 is largely due to an effect of improving the efficiency of altering a DNA by a UGI (it is known that the effects of UGI are significantly manifested in yeast), and a complex that can achieve the desired efficiency of alteration cannot be obtained simply by deleting a terminal region of CDA1 when a UGI is not used, or when applied to other organism species.

In this regard, the conventional concept of simply deleting a terminal region of a cytidine deaminase was reconsidered to arrive at a hypothesis that carefully studying the structure of a cytidine deaminase and altering the structure based on said structure may prevent a decrease in the efficiency of altering a target site. As a result of conducting a study based on this hypothesis, the N-terminal side and the C-terminal side were simultaneously deleted while taking into consideration the interaction inside a cytidine deaminase to approximate the three-dimensional structure of the cytidine deaminase to a spherical shape, and an exposed internal amino acid residue was replaced from a hydrophobic to hydrophilic amino acid residue to successfully materialize stabilization of the cytidine deaminase and recover the efficiency. Furthermore, efficiency beyond that of conventional technologies was also achieved by improving the stability as a complex and improving access to a substrate DNA by embedding CDA1, which was fused to a terminus of Cas9 in conventional technologies, inside Cas9. When an off-target effect was evaluated, it was found that an off-target effect can be suppressed more significantly in each case compared to conventional technologies. Further, CRISPR-Cas with a size that can be inserted in an AAV vector was materialized by using SaCas9 as Cas9. The inventors completed the present disclosure as a result of further studies based on these findings.

Therefore, the present disclosure provides the following.

### (Item 1)

A complex of a nucleic acid sequence recognition module bound with a deaminase, wherein
the nucleic acid sequence recognition module specifically binds to a target nucleotide sequence in a double stranded DNA,
the deaminase is altered so that the deaminase has a smaller size than a wild-type deaminase corresponding to the deaminase, and an area of a cross-section exposed as a result of alteration or an index indicating the area is less than or equal to a predetermined value, and
the complex has an ability to alter a targeted site of the double stranded DNA.

### (Item 2)

The complex of the preceding item, wherein the deaminase is altered so that the number of hydrophobic amino acid residues manifested on a cross-section exposed as a result of altering the deaminase is less than or equal to a predetermined value, and the alteration comprises a deletion.

### (Item 3)

The complex of any one of the preceding items, wherein the deaminase is altered so that a ratio of the number of hydrophobic residues manifested on a cross-section exposed as a result of alteration to the number of altered amino acid residues is less than or equal to a predetermined value, and the alteration comprises a deletion.

### (Item 4)

The complex of any one of the preceding items, wherein the deaminase is altered so that the number of hydrophobic amino acid residues manifested on a cross-section exposed as a result of altering the deaminase is minimized.

### (Item 5)

The complex of any one of the preceding items, wherein the deaminase is altered so that a ratio of the number of hydrophobic residues manifested on a cross-section exposed as a result of alteration to the number of altered amino acid residues is minimized.

### (Item 6)

The complex of any one of the preceding items, wherein the deaminase is from altering an N-terminus side and a C-terminus side of the wild-type deaminase.

### (Item 7)

The complex of any one of the preceding items, wherein at least one hydrophobic internal amino acid residue exposed in the deaminase is substituted with a hydrophilic amino acid residue.

### (Item 8)

The complex of any one of the preceding items, wherein the deaminase comprises a cytidine deaminase.

### (Item 9)

The complex of any one of the preceding items, wherein the deaminase consists of:
(1) an amino acid sequence consisting of a region of amino acid residues at positions 30 to 150 in the amino acid sequence set forth in SEQ ID NO: 1;
(2) an amino acid sequence consisting of a region corresponding to the region of (1) which is an ortholog of a protein consisting of the amino acid sequence set forth in SEQ ID NO: 1;
(3) the amino acid sequence of (1) or (2) with one or several amino acid deletions, substitutions, insertions, and/or additions; or
(4) an amino acid sequence with 90% or greater similarity or identity with the amino acid sequence of (1) or (2).

### (Item 10)

The complex of any one of the preceding items, wherein the amino acid sequence of (3) comprises one or more substitutions of an amino acid residue at a position selected from the group consisting of positions 122, 126, and 139 in the amino acid sequence set forth in SEQ ID NO: 1 or an amino acid residue corresponding to said position to a hydrophilic amino acid residue.

### (Item 11)

The complex of any one of the preceding items, wherein the amino acid sequence of (3) comprises two or more substitutions of an amino acid residue at position 122 and an amino acid residue at position 139 in the amino acid sequence set forth in SEQ ID NO: 1 or amino acid residues corresponding to said positions to hydrophilic amino acid residues.

### (Item 12)

The complex of any one of the preceding items, wherein the nucleic acid sequence recognition module is selected from the group consisting of a CRISPR-Cas system wherein at least one DNA cleaving capability of a Cas protein is inactivated, a zinc finger motif, a TAL effector, and a PPR motif.

### (Item 13)

The complex of any one of the preceding items, wherein the nucleic acid sequence recognition module is a CRISPR-Cas system wherein at least one DNA cleaving capability of a Cas protein is inactivated.

### (Item 14)

The complex of any one of the preceding items, wherein the Cas protein is a Cas9 protein.

### (Item 15)

A complex of an N-terminal fragment of a nucleic acid sequence recognition module, a deaminase, and a C-terminal fragment of a nucleic acid sequence recognition module bound therewith, wherein
when the N-terminal fragment and the C-terminal fragment of the nucleic acid sequence recognition module are refolded, the nucleic acid sequence recognition module has an ability to specifically bind to a target nucleotide sequence in a double stranded DNA and alter a targeted site of the double stranded DNA.

### (Item 16)

The complex of the preceding item, wherein the deaminase is altered so that the deaminase has a smaller size than a wild-type deaminase corresponding to the deaminase, and an area of a cross-section exposed as a result of alteration or an index indicating the area is less than or equal to a predetermined value.

### (Item 17)

The complex of any one of the preceding items, wherein the deaminase is altered so that the number of hydrophobic amino acid residues manifested on a cross-section exposed as a result of altering the deaminase is less than or equal to a predetermined value, and the alteration comprises a deletion.

### (Item 18)

The complex of any one of the preceding items, wherein the deaminase is altered so that a ratio of the number of hydrophobic residues manifested on a cross-section exposed as a result of alteration to the number of altered amino acid residues is less than or equal to a predetermined value, and the alteration comprises a deletion.

### (Item 19)

The complex of any one of the preceding items, wherein the deaminase is altered so that the number of hydrophobic amino acid residues manifested on a cross-section exposed as a result of altering the deaminase is minimized.

### (Item 20)

The complex of any one of the preceding items, wherein the deaminase is altered so that a ratio of the number of hydrophobic residues manifested on a cross-section exposed as a result of alteration to the number of altered amino acid residues is minimized.

### (Item 21)

The complex of any one of the preceding items, wherein the deaminase is from altering an N-terminus side and a C-terminus side of the wild-type deaminase.

### (Item 22)

The complex of any one of the preceding items, wherein at least one hydrophobic internal amino acid residue exposed in the deaminase is substituted with a hydrophilic amino acid residue.

### (Item 23)

The complex of any one of the preceding items, wherein the deaminase comprises a cytidine deaminase.

### (Item 24)

The complex of any one of the preceding items, wherein the deaminase consists of:
(1) an amino acid sequence consisting of a region of amino acid residues at positions 30 to 150 in the amino acid sequence set forth in SEQ ID NO: 1;
(2) an amino acid sequence consisting of a region corresponding to the region of (1) which is an ortholog of a protein consisting of the amino acid sequence set forth in SEQ ID NO: 1;
(3) the amino acid sequence of (1) or (2) with one or several amino acid deletions, substitutions, insertions, and/or additions; or
(4) an amino acid sequence with 90% or greater similarity or identity with the amino acid sequence of (1) or (2).

### (Item 25)

The complex of any one of the preceding items, wherein the nucleic acid sequence recognition module is selected from the group consisting of a CRISPR-Cas system wherein at least one DNA cleaving capability of a Cas protein is inactivated, a zinc finger motif, a TAL effector, and a PPR motif.

### (Item 26)

A nucleic acid encoding the complex of any one of the preceding items.

### (Item 27)

A vector comprising the nucleic acid of the preceding item.

### (Item 28)

The vector of the preceding item, which is an adeno-associated viral vector.

### (Item 29)

A method of altering a targeted site of a double stranded DNA of a cell, comprising contacting the complex of any one of the preceding items with the double stranded DNA.

### (Item 30)

The method of the preceding item, wherein contacting a double stranded DNA with a complex is performed through introduction of the nucleic acid or vector of any one of the preceding items into the cell.

The present disclosure also provides the following.
[1] A complex of a nucleic acid sequence recognition module bound with a cytidine deaminase, wherein
   the nucleic acid sequence recognition module specifically binds to a target nucleotide sequence in a double stranded DNA,
   the cytidine deaminase consists of:
      (1) an amino acid sequence consisting of a region of amino acid residues at positions 30 to 150 in the amino acid sequence set forth in SEQ ID NO: 1;
      (2) an amino acid sequence consisting of a region corresponding to the region of (1) which is an ortholog of a protein consisting of the amino acid sequence set forth in SEQ ID NO: 1;
      (3) the amino acid sequence of (1) or (2) with one or several amino acid deletions, substitutions, insertions, and/or additions; or
      (4) an amino acid sequence with 90% or greater similarity or identity with the amino acid sequence of (1) or (2), and
   the complex alters a targeted site of the double stranded DNA.
[2] The complex of [1], wherein the amino acid sequence of (3) comprises one or more substitutions of an amino acid residue at a position selected from the group consisting of positions 122, 126, and 139 in the amino acid sequence set forth in SEQ ID NO: 1 or an amino acid residue corresponding to said position to a hydrophilic amino acid residue.
[3] The complex of [1] or [2], wherein the amino acid sequence of (3) comprises two or more substitutions of an amino acid residue at position 122 and an amino acid residue at position 139 in the amino acid sequence set forth in SEQ ID NO: 1 or amino acid residues corresponding to said positions to hydrophilic amino acid residues.
[4] The complex of any one of [1] to [3], wherein the nucleic acid sequence recognition module is selected from the group consisting of a CRISPR-Cas system wherein at least one DNA cleaving capability of a Cas protein is inactivated, a zinc finger motif, a TAL effector, and a PPR motif.
[5] The complex of any one of [1] to [3], wherein the nucleic acid sequence recognition module is a CRISPR-Cas system wherein at least one DNA cleaving capability of a Cas protein is inactivated.
[6] The complex of [4] or [5], wherein the Cas protein is a Cas9 protein.
[7] A complex of an N-terminal fragment of a nucleic acid sequence recognition module, a cytidine deaminase, and a C-terminal fragment of a nucleic acid sequence recognition module bound therewith, wherein
   when the N-terminal fragment and the C-terminal fragment of the nucleic acid sequence recognition module are refolded, the nucleic acid sequence recognition module specifically binds to a target nucleotide sequence in a double stranded DNA and alters a targeted site of the double stranded DNA.
[8] The complex of [7], wherein the cytidine deaminase consists of:
   (1) an amino acid sequence consisting of a region of amino acid residues at positions 30 to 150 in the amino acid sequence set forth in SEQ ID NO: 1;
   (2) an amino acid sequence consisting of a region corresponding to the region of (1) which is an ortholog of a protein consisting of the amino acid sequence set forth in SEQ ID NO: 1;
   (3) the amino acid sequence of (1) or (2) with one or several amino acid deletions, substitutions, insertions, and/or additions; or
   (4) an amino acid sequence with 90% or greater similarity or identity with the amino acid sequence of (1) or (2).
[9] The complex of [7] or [8], wherein the nucleic acid sequence recognition module is selected from the group consisting of a CRISPR-Cas system wherein at least one DNA cleaving capability of a Cas protein is inactivated, a zinc finger motif, a TAL effector, and a PPR motif.
[10] A nucleic acid encoding the complex of any one of [1] to [9] .
[11] A vector comprising the nucleic acid of [10].
[12] The vector of [11], which is an adeno-associated viral vector.
[13] A method of altering a targeted site of a double stranded DNA of a cell, comprising contacting the complex of any one of [1] to [9] with the double stranded DNA.
[14] The method of [13], wherein contacting a double stranded DNA with a complex is performed through introduction of the nucleic acid or vector of any one of [10] to [12] into the cell.

### [Advantageous Effects of Invention]

The present disclosure provides a complex for altering a double stranded DNA, which is more compact, has higher efficiency of alteration, and has an off-target effect that is more suppressed compared to conventional technologies. A targeted site of a DNA can be altered more safely without cleaving a double stranded DNA by using such a complex. A nucleic acid encoding such a complex can also be inserted into an adeno-associated viral vector and facilitate delivery of the complex to a target site, so that such a nucleic acid can be useful especially for application to gene therapy, etc.

### [Brief Description of Drawings]

[Figure 1] Figure **1** shows results of aligning the sequences of human AID (denoted as HsAID), wild-type PmCDA1, and one embodiment of the deaminase of the present disclosure (PmCDA1-36) . The sequence of HsAID is set forth in SEQ ID NO: 3, the sequence of wild-type PmCDA1 is set forth in SEQ ID NO: 1, and the sequence of PmCDA1-36 is set forth in SEQ ID NO: 2.
[Figure 2] Figure **2** is a schematic diagram of the plasmid constructs used in Example 1. The number in each construct indicates the position of the amino acid of a protein encoded by each sequence.
[Figure 3] Figure **3** shows results of Example 1. -Canavanine indicates a canavanine-free medium, and +Canavanine indicates a canavanine-containing medium.
[Figure 4] Figure **4** shows results of Example 1. -Canavanine indicates a canavanine-free medium, and +Canavanine indicates a canavanine-containing medium.
[Figure 5] Figure **5** shows a graph of results in Example 1. The vertical axis indicates the mutation introduction rate (%). The rightmost KN1251 is a positive control.
[Figure 6] Figure **6** shows results of three-dimensional structure analysis on CDA1. The left diagram shows the three-dimensional structure of wild-type CDA1, and the right diagram shows the three dimensional structure of CDA1Δ161.
[Figure 7] Figure **7** shows the exposed internal amino acid residues of CDA1Δ161 (white portion in the right diagram).
[Figure 8] Figure **8** is a schematic diagram of plasmid constructs used in Example 2. The number in each construct indicates the position of the amino acid of a protein encoded by each sequence.
[Figure 9] Figure **9** shows results of Example 2. -Canavanine indicates a canavanine-free medium, and +Canavanine indicates a canavanine-containing medium.
[Figure 10] Figure **10** shows results of Example 2. -Canavanine indicates a canavanine-free medium, and +Canavanine indicates a canavanine-containing medium.
[Figure 11] Figure **11** shows a graph of results in Example 2. The vertical axis indicates the mutation introduction rate (%). The rightmost KN1252 is a positive control.
[Figure 12] Figure **12** is a schematic diagram of plasmid constructs used in Example 2. The number in each construct indicates the position of the amino acid of a protein encoded by each sequence.
[Figure 13] Figure **13** shows the portion introduced with a mutation (white portion) in the three dimensional structure of Example 2.
[Figure 14] Figure **14** shows results of Example 2. -Canavanine indicates a canavanine-free medium, and +Canavanine indicates a canavanine-containing medium.
[Figure 15] Figure **15** shows results of Example 2. -Canavanine indicates a canavanine-free medium, and +Canavanine indicates a canavanine-containing medium.
[Figure 16] Figure **16** shows a graph of results in Example 2. The vertical axis indicates the mutation introduction rate (%). The rightmost KN1252 is a positive control.
[Figure 17] Figure **17** is a schematic diagram of plasmid constructs used in Example 2. The number in each construct indicates the position of the amino acid of a protein encoded by each sequence.
[Figure 18] Figure **18** shows results of Example 2. -Canavanine indicates a canavanine-free medium, and +Canavanine indicates a canavanine-containing medium.
[Figure 19] Figure **19** shows results of Example 2. -Canavanine indicates a canavanine-free medium, and +Canavanine indicates a canavanine-containing medium.
[Figure 20] Figure **20** shows a graph of results in Example 2. The target sequence of a guide RNA differs in the left and right diagrams. The vertical axis of each diagram indicates the mutation introduction rate (%). The rightmost KN1252 of each diagram is a positive control.
[Figure 21] Figure **21** is a schematic diagram of plasmid constructs used in Example 3. The number in each construct indicates the position of the amino acid of a protein encoded by each sequence.
[Figure 22] Figure **22** shows results of Example 3. -Canavanine indicates a canavanine-free medium, and +Canavanine indicates a canavanine-containing medium.
[Figure 23] Figure **23** shows results of Example 3. -Canavanine indicates a canavanine-free medium, and +Canavanine indicates a canavanine-containing medium.
[Figure 24] Figure **24** shows a graph of results in Example 3. The target sequence of a guide RNA differs in the left and right diagrams. The vertical axis of each diagram indicates the mutation introduction rate (%). The rightmost KN1252 of each diagram is a positive control.
[Figure 25] Figure **25** shows a schematic diagram of plasmid constructs used in Example 3, and a tertiary structure of one embodiment of the deaminase of the present disclosure. The number in each construct indicates the position of the amino acid of a protein encoded by each sequence.
[Figure 26] Figure **26** shows a schematic diagram of plasmid constructs used in Example 3. The number in each construct indicates the position of the amino acid of a protein encoded by each sequence.
[Figure 27] Figure **27** shows results of Example 3. -Canavanine indicates a canavanine-free medium, and +Canavanine indicates a canavanine-containing medium.
[Figure 28] Figure **28** shows results of Example 3. -Canavanine indicates a canavanine-free medium, and +Canavanine indicates a canavanine-containing medium.
[Figure 29] Figure **29** shows a graph of results in Example 3. The target sequence of a guide RNA differs in the left and right diagrams. The vertical axis of each diagram indicates the mutation introduction rate (%). The rightmost KN1252 of each diagram is a positive control.
[Figure 30] Figure **30** shows results of Example 3. -Canavanine indicates a canavanine-free medium, and +Canavanine indicates a canavanine-containing medium.
[Figure 31] Figure **31** shows results of Example 3. -Canavanine indicates a canavanine-free medium, and +Canavanine indicates a canavanine-containing medium.
[Figure 32] Figure **32** shows a graph of results in Example 3. The target sequence of a guide RNA differs in the left and right diagrams. The vertical axis of each diagram indicates the mutation introduction rate (%). The rightmost KN1252 of each diagram is a positive control.
[Figure 33] Figure **33** shows a schematic diagram of plasmid constructs used in Example 4. The number in each construct indicates the position of the amino acid of a protein encoded by each sequence.
[Figure 34] Figure **34** shows results of Example 4. -Canavanine indicates a canavanine-free medium, and +Canavanine indicates a canavanine-containing medium.
[Figure 35] Figure **35** shows results of Example 4. -Canavanine indicates a canavanine-free medium, and +Canavanine indicates a canavanine-containing medium.
[Figure 36] Figure **36** shows results of Example 5.
[Figure 37] Figure **37** shows results of Example 5.
[Figure 38] Figure **38** shows results of Example 5.
[Figure 39] Figure **39** shows results of Example 5.
[Figure 40] Figure **40** shows results of Example 5.
[Figure 41] Figure **41** shows results of Example 5.
[Figure 42] Figure **42** shows a schematic diagram of plasmid constructs used in Example 6. The number in each construct indicates the position of the amino acid of a protein encoded by each sequence, but those appended with bp indicate the nucleotide length.
[Figure 43] Figure **43** is a schematic diagram of a plasmid encoding a guide RNA (top diagram) and a schematic diagram of the procedure of the experiment in Example 6 (bottom diagram).
[Figure 44] Figure **44** shows results of Example 6. Mutation efficiency and the nucleotide after a mutation in each nucleotide of a sequence are shown.
[Figure 45] Figure **45** shows a graph of results of Example 6. The vertical axis corresponds mutation efficiency, and the horizontal axis corresponds to the numerical value in the 24 well section and the sequence in the Reference section of Figure **44****.** The sequence described in the top left graph is set forth in SEQ ID NO: 17, the sequence described in the bottom left graph is set forth in SEQ ID NO: 18, the sequence described in the top right graph is set forth in SEQ ID NO: 19, and the sequence described in the bottom right graph is set forth in SEQ ID NO: 20.

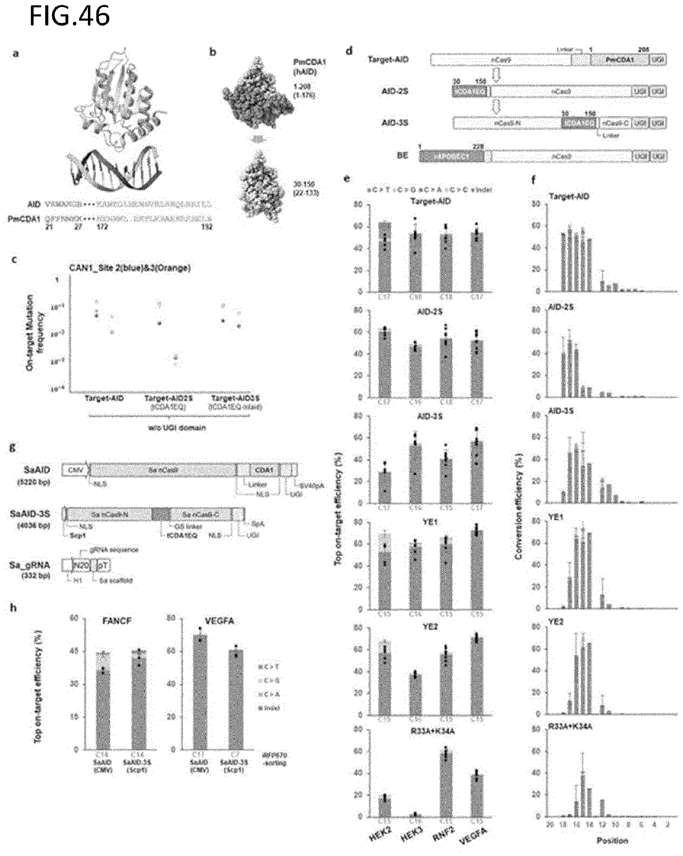

[Figure 46] Figure **46a** is a ribbon model showing the structure of a complex of human AID and dsDNA. Non-catalytic double stranded DNA binding domains are shown in green (N-terminus) and red (C-terminus). The amino acid sequence thereof is compared with that of PmCDA1 below. In Figure **46a****,** the sequences shown in AID are set forth in, in order from the left, SEQ ID NO: 21 and SEQ ID NO: 22. The sequences shown in PmCDA1 are set forth in, in order from the left, SEQ ID NO: 23 and SEQ ID NO: 24. Figure **46b** is a predicted diagram of honeycomb structures before and after alteration of PmCDA1. In addition to the direct DNA binding sites (red and green), the segment shown in blue was trimmed to minimize the cross-section of the protein. Mutated amino acids (W122 and W139) are shown in yellow. Figure **46c** is a graph showing on-target editing efficiency of Target-AID, AID-2S, and AID-33 free of a UGI in a canavanine resistance assay of yeasts. CAN1-2 (blue points) and CAN1-3 (orange points) were chosen as target sites, and biological triple structures were plotted. Figure **46d** is a schematic diagram showing the domain arrangement of a CBE mutant used in the Example. The structure of BE is shared by YE1, YE2, and R33A+K34A except for a point mutation of rAPOBEC1. Figures **46e** and **46f** are graphs showing the on-target editing profile of a CBE variant analyzed by deep sequencing at HEK2, HEK3, RNF2, and VEGFA sites of HEK293T. Figure **46e** shows nucleotide positions with the highest frequency of C→T conversion of each target (numbers were assigned from the PAM sequence side of a target sequence toward the 5' side). Figure **46f** shows the average editing window of four targets. Figures **46e**, f, and h show the mean score (rectangular bar) and standard deviation (error bar). If n < 9, each biological replication was plotted as a dot. Figure **46g** is a schematic diagram showing the domain structures of SaAID and SaAID-3S. A gRNA expression cassette is bound to each effector plasmid. Figure **46h** is a graph showing the on-target editing frequency of SaAID and SaAID-3S in HEK293T in the same manner as Figure **46e****.** Cells were sorted out by expression of iRFP670 from the plasmid backbone in order to normalize the transfection efficiency.

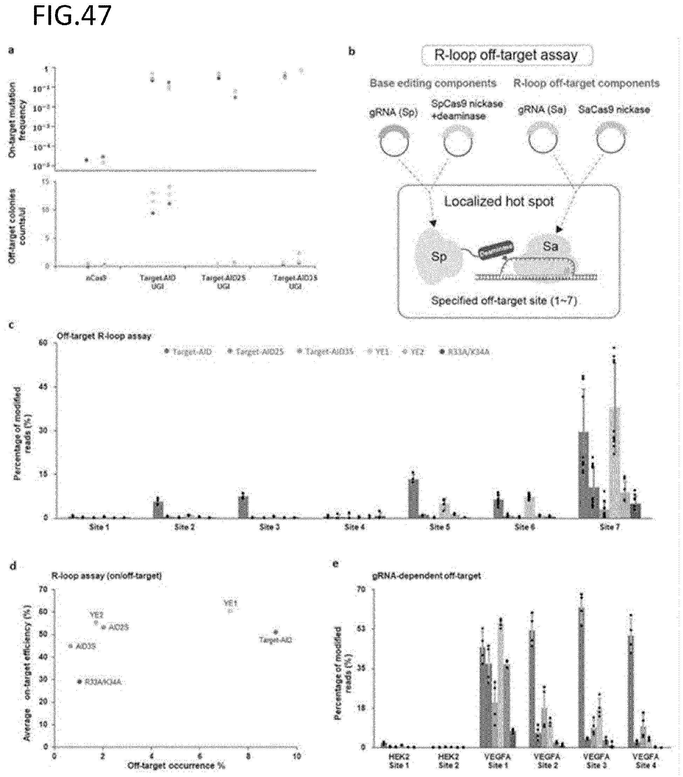

[Figure 47] Figure **47a** is a graph of measuring the rate of occurrence of on-target mutation (canavanine resistant) and off-target mutation (thialysine resistant) after inducing each construct (AID-2S, -3S, and rAPOBEC1) as shown in yeasts. Values of biological repeats were plotted for a target site of CAN1-2 (blue points) and CAN1-3 (orange points). Figure **47b** is a schematic diagram of an orthogonal R-loop off-target assay. Figure **47c** is a graph showing results of selecting seven off-target R-loop sites (1 to 7), co-introducing with one of the on-target sites (HEK2, HEK3, RNF2, and VEGFA), and analyzing with a deep sequencer. The off-target frequency is indicated by the ratio of reads comprising a mutation. For sites 1, 2 to 5, 6, and 7, data sets of n = 6, n = 4, n = 12, and n = 10, respectively, were plotted. The average frequency (rectangular bar) and standard deviation (error bar) thereof are shown. Figure **47d** is a graph showing an on-target editing to average off-target editing profile of all CBEs in the Example. The y-axis represents the average on-target editing of four on-target sites (HEK2, HEK3, RNF2, and VEGFA) used in an R-loop assay, and the x-axis represents the average off-target editing of seven orthogonal R-loop sites. Figure **47e** is a graph of evaluating Cas9 dependent off-target effects. Two HEK2 off-target sites (1 to 2) and four VEGFA off-target sites (1 to 4) were analyzed by deep sequencing. The data set was n = 4.

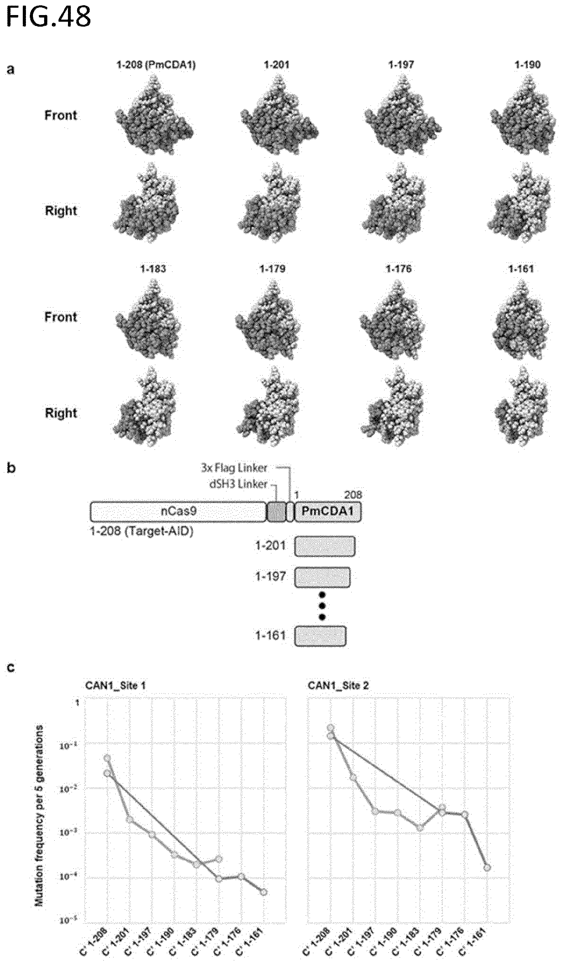

[Figure 48] Figure **48** is a diagram showing the effect from deleting the C-terminus of PmCDA1. Figure **48a** shows predicted diagrams of a series of honeycomb structures of PmCDA1 with a deletion of the C-terminus. The dsDNA binding domains without catalytic action are shown in green (N-terminus) and red (C-terminus). Figure **48b** is a schematic diagram showing the C-terminus cleaved Target-AID constructs investigated in Figure **48c.** Figure **48c** is a graph showing the change in on-target editing efficiency of a cleaved construct in yeasts. The rate of occurrence of canavanine resistant mutants was measured as a CAN1 gene mutant. For CAN1-1 and CAN1-2 target sites, trend lines of different data sets (pink and gray dots) were plotted.

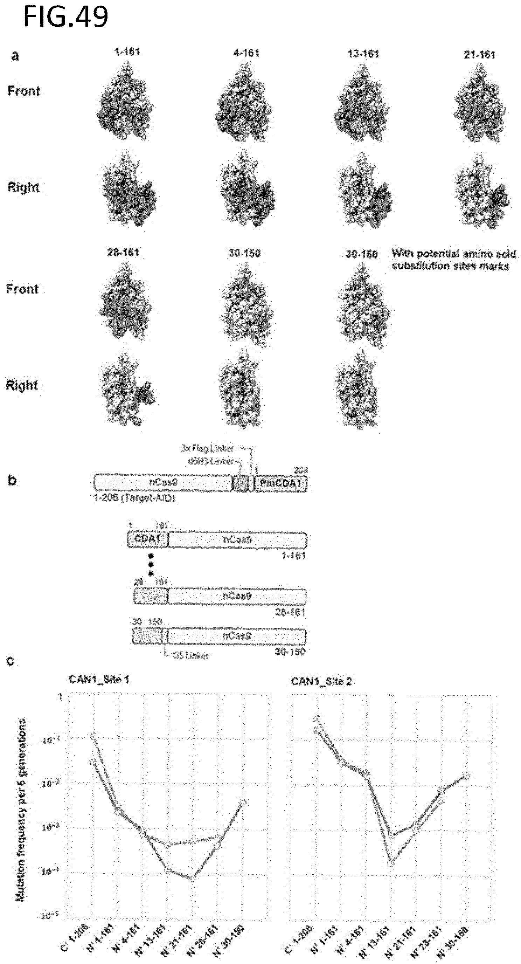

[Figure 49] Figure **49** is a diagram showing the effect from deleting the N-terminus and C-terminus of PmCDA1. Figure **49a** shows predicted diagrams of a series of honeycomb structures of PmCDA1 with deletion of the N-terminus and C-terminus. The dsDNA binding domains without catalytic action are shown in green (N-terminus). The blue segments indicate adjacent sites cleaved to smooth out the shape of a protein and minimize the cross-section. Figure **49b** is a schematic diagram showing the N-terminus and C-terminus cleaved Target-AID construct tested in Figure **49c.** Figure **49c** is a graph showing the trend in on-target editing efficiency of a cleaved construct in yeasts. The rate of occurrence of canavanine resistant mutants was measured as a CAN1 gene mutant. For CAN1-1 and CAN1-2 target sites, trend lines of different data sets (pink and gray dots) were plotted.
[Figure 50] Figure **50** is a diagram showing the effect of amino acid substitution in cleaved PmCDA1 (30-150). Hydrophobic residues exposed after cleavage were substituted with a hydrophilic residue. On-target editing efficiency was measured by a yeast canavanine assay as shown in Figure **49** and normalized with an average value of Target-AID. Biological replications were plotted for CAN1-1 (red) and CAN1-2 (blue) target sites. Different data sets are indicated by different shapes of dots.
[Figure 51] Figure **51** is a diagram showing domain embedded Target-AID3S. Figure **51a** is a schematic diagram showing domain embedded AID-3S (1054-tCDA1EQ-1055) at the position of the RuvC domain of Cas9. Figure **51b** shows on-target editing efficiency evaluated in yeasts. For CAN1-2 (blue) and CAN1-3 (orange) target sites, the values of biological repeats were plotted.
[Figure 52] Figure **52** is a diagram showing the on-target editing profiles of SaCas9-AID mutants. On-target editing performance evaluated with HEKT293 cells using iRFP670 cell sorting is shown. FANCF and VEGFA target sites were chosen, and the frequency of mutation at each nucleotide position is shown. The average score (rectangular bar) and standard deviation (error bar) of each nucleotide conversion are shown. Each biological replication is plotted as a point (n = 3).

### [Description of Embodiments]

The present disclosure is described hereinafter while showing the best mode thereof.

Throughout the entire specification, a singular expression should be understood as encompassing the concept thereof in the plural form, unless specifically noted otherwise. Thus, singular articles (e.g., "a", "an", "the", and the like in the case of English) should also be understood as encompassing the concept thereof in the plural form, unless specifically noted otherwise. Further, the terms used herein should be understood as being used in the meaning that is commonly used in the art, unless specifically noted otherwise. Therefore, unless defined otherwise, all terminologies and scientific technical terms that are used herein have the same meaning as the general understanding of those skilled in the art to which the present disclosure pertains. In case of a contradiction, the present specification (including the definitions) takes precedence.

As used herein, "about" refers to a range of ± 10% from the numerical value that is described subsequent to "about".

### 1. Nucleic acid altering enzyme complex

One aspect of the present disclosure provides a complex of a nucleic acid sequence recognition module bound with a deaminase, wherein the nucleic acid sequence recognition module specifically binds to a target nucleotide sequence in a double stranded DNA, the deaminase is altered so that the deaminase has a smaller size than a wild-type deaminase corresponding to the deaminase, and an area of a cross-section exposed as a result of alteration or an index indicating the area is less than or equal to a predetermined value, and the complex has an ability to alter a targeted site of the double stranded DNA. In one embodiment, an area of a cross-section exposed as a result of alteration or an index indicating the area can be less than or equal to a predetermined value appropriately determined in accordance with the type of deaminase. For example, the exposed cross-section index or the number of hydrophobic amino acid residues described below can be used as such an index.

The objective of the present disclosure is to provide a base editing system that can be inserted into a single AAV vector by making a deaminase compact. The inventors discovered that reducing or preferably minimizing the area of a cross-section exposed when an amino acid has been altered by deletion, etc. or an index indicating the area to be less than or equal to a predetermined value, when making a deaminase compact, leads to stabilization of the structure. An element specifically considered important is the number of hydrophobic amino acids manifested on a cross-section exposed when amino acids are deleted or substituted to be less than or equal to a predetermined value, e.g., reducing or preferably minimizing the number to less than the number from applying other alterations.

In one embodiment of the present disclosure, a deaminase can be altered on the N-terminus side and C-terminus side of a wild-type deaminase, so that the number of hydrophobic amino acid residues manifested on a cross-section exposed as a result of altering the deaminase is less than or equal to a predetermined value, or minimized. In another embodiment, amino acid residues of an altered deaminase are not limited to the N-terminus or C-terminus. Amino acids inside the sequence (not at a terminal) can be altered, as long as the alteration results in the number of hydrophobic amino acid residues manifested on a cross-section exposed as a result of altering the deaminase to be less than or equal to a predetermined value, or minimized.

If it is desirable to simply minimize the number of amino acids exposed by a deletion or substitution when altering a deaminase, avoiding extensive alteration is sufficient. Alternatively, the ratio of: hydrophobic residues exposed, to altered amino acids can be found and the numerical value thereof (also referred to as an "exposed cross-section index" herein) can be used as an indicator in order make a deaminase compact and minimize hydrophobic amino acids manifested on a cross-section exposed when amino acids are altered. Thus, in one embodiment, the deaminase can be altered so that a ratio of: the number of hydrophobic amino acid residues manifested on a cross-section exposed as a result of alteration, to the number of altered amino acid residues is less than or equal to a predetermined ratio or minimized. The indicator can also be reduced by substituting a hydrophobic residue manifested on a cross-section exposed as a result of alteration with a hydrophilic residue. Thus, in one embodiment, at least one hydrophobic internal amino acid residue exposed in a deaminase can be substituted with a hydrophilic amino acid residue.

As described in detail in the Examples presented below, 14 hydrophobic residues (Y34, L36, F49, W50, Y52, Y78, Y91, L105, W122, L126, Y128, 1136, W139, and V150) are manifested for a compact cytidine deaminase PmCDA1 (30-150). The ratio of: the number of hydrophobic residues exposed (14 residues), to the number of deleted amino acids (87 residues) can be calculated to quantify the exposure cross-section index.

Thus, in one embodiment, the ratio of: the number of hydrophobic residues manifested on a cross-section exposed as a result of alteration, to the number of altered amino acid residues can be, for example, about 3% or less, about 4% or less, about 5% or less, about 6% or less, about 7% or less, about 8% or less, about 9% or less, about 10% or less, about 12% or less, about 14% or less, about 16% or less, about 18% or less, about 20% or less, about 22% or less, about 24% or less, about 26% or less, about 28% or less, about 30% or less, about 35% or less, about 40% or less, about 45% or less, about 50% or less, about 55% or less, about 60% or less, about 65% or less, about 70% or less, about 75% or less, about 80% or less, about 85% or less, about 90% or less, or about 95% or less.

In one embodiment, the number of hydrophobic amino acid residues manifested on a cross-section exposed as a result of altering the deaminase can be, for example, about 1 or less, about 2 or less, about 3 or less, about 4 or less, about 5 or less, about 6 or less, about 7 or less, about 8 or less, about 9 or less, about 10 or less, about 11 or less, about 12 or less, about 13 or less, about 14 or less, about 15 or less, about 16 or less, about 17 or less, about 18 or less, about 19 or less, about 20 or less, about 22 or less, about 24 or less, about 26 or less, about 28 or less, about 30 or less, about 35 or less, about 40 or less, about 45 or less, about 50 or less, about 55 or less, about 60 or less, about 65 or less, about 70 or less, about 75 or less, about 80 or less, about 85 or less, about 90 or less, about 95 or less, or about 100 or less. In another embodiment, an alteration may result in the number of hydrophobic amino acid residues manifested on a cross-section exposed as a result of altering the deaminase to be 100 or more, if the alteration results in the ratio of: the number of hydrophobic residues manifested on a cross-section exposed as a result of alteration, to the number of altered amino acid residues described above that is less than or equal to a predetermined value.

As used herein, "size" refers to the physical or chemical size of a molecule such as a protein, including sizes of the molecular weight, occupied volume, mass, etc. A decrease in size includes reduction of the molecular weight, volume, mass, etc. of the molecule. Preferably, the molecular weight can be a more suitable indicator.

As used herein, "minimize" refers to a value that is at least decreased or smaller compared to the state prior to alteration or compared to when another alteration is administered. The value does not need to be the minimum value.

As used herein, "alteration" includes deletion or substitution of an amino acid.

Whether a specific amino acid within a protein (deaminase, etc.) is exposed when altered can be accurately calculated by modeling, etc. For example, the structure of a protein can be predicted by referring to I-TASSER (https://zhanggroup.org/I-TASSER/). The original protein structure for predicting the structure can be obtained from, for example, RCSBPDB (https://www.rcsb.org/), etc. For AID, 5W1C (https://www.rcsb.org/structure/5W1C) can be used.

In one embodiment, the present disclosure provides a complex of a nucleic acid sequence recognition module that specifically binds to a target nucleotide sequence in the double stranded DNA bound with a compact cytidine deaminase, for altering a double stranded DNA (also referred to as the "compact complex of the present disclosure" hereinafter). As described in the following section 3, a targeted site of a double stranded DNA of interest (e.g., genomic DNA) can be altered by contacting the compact complex of the present disclosure with the double stranded DNA.

As discussed in the Examples presented below, a cytidine deaminase, which was fused to a terminus of Cas9 in the past, is embedded inside a Cas effector protein to improve the stability as a complex and access to substrate DNA and achieve a higher efficiency than using a conventional complex. Thus, another embodiment of the present disclosure provides a complex of an N-terminal fragment of a nucleic acid sequence recognition module, a cytidine deaminase, and a C-terminal fragment of a nucleic acid sequence recognition module bound therewith (hereinafter, also referred to as the "complex of the present disclosure (split form) ") . A cytidine deaminase constituting the complex of the present disclosure (split form), even wild-type cytidine deaminase, achieve high efficiency of alteration, but a compact cytidine deaminase is preferable from the viewpoint of achieving a compact size or suppression of an off-target effect. In the complex of the present disclosure (split form), refolding of an N-terminal fragment and a C-terminal fragment of a nucleic acid sequence recognition module enables the nucleic acid sequence recognition module to specifically bind to a target nucleotide sequence in the double stranded DNA. Hereinafter, the term "complex of the present disclosure" may be used as a term encompassing both the "compact complex of the present disclosure" and "complex of the present disclosure (split form)".

The complex of the present disclosure (split form) may be provided as a fusion protein comprising an N-terminal fragment of a nucleic acid sequence recognition module, a cytidine deaminase, and a C-terminal fragment of a nucleic acid sequence recognition module from the N-terminus to the C-terminus, in order or in reverse order. In such a case, at least one of the elements may be linked via a suitable linker (e.g., 3xFlag linker, GS linker, etc.) or bound without a linker. Alternatively, a split enzyme can be used, which is designed to reconstitute a functional cytidine deaminase when each of a nucleic acid sequence recognition module and a cytidine deaminase is split into two fragments and respective fragments thereof are linked to each other to form two partial complexes, then the complexes are associated, and a functional nucleic acid sequence recognition module is reconstituted and binds to a target nucleotide sequence. For example, a functional nucleic acid sequence recognition module and a functional cytidine deaminase can be reconstituted by splitting each of a nucleic acid sequence recognition module and a cytidine deaminase into an N-terminus side fragment and a C-terminus side fragment, preparing, for example, a partial complex from linking N-terminus side fragments with each other and a partial complex from linking C-terminus side fragments with each other, and associating the complexes. The two partial complexes may also be provided as separate molecules or as a single fusion protein by linking the complexes directly or via a suitable linker.

The Examples presented below have demonstrated that the same or higher efficiency of alteration than from using a conventional complex can be achieved when a cytidine deaminase was inserted at a plurality of positions in the amino acid sequence of a nucleic acid sequence recognition module in the complex of the present disclosure (split form), at any position. It was also demonstrated that a mutation introduction site can be adjusted by adjusting the position of insertion. Thus, the position where a cytidine deaminase is inserted is not particularly limited. For example, it is preferable to split SpCas9 between an amino acid residue of any of positions 204 to 1054 (e.g., position 204, 535, 1023, or 1054) of SpCas9 (SEQ ID NO: 4) and an amino acid residue at a position offset by one amino residue to the C-terminus side (e.g., position 205, 536, 1024, or 1055) when using a CRISPR-SpCas9 system as a nucleic acid sequence recognition module. When inserting a cytidine deaminase between, for example, amino acid residues at positions 204 and 205 of SpCas9, the C-terminal fragment of SpCas9 would be a fragment consisting of 1 to 204, and the N-terminal fragment would be a fragment consisting of 205 to 1368. It is preferable to split SaCas9 between an amino acid residue of any of positions 127 to 848 (e.g., position 127, 538, 614, 690, 735, or 848) of SaCas9 (SEQ ID NO: 5) and an amino acid residue at a position offset by one amino residue to the C-terminus side (e.g., position 128, 539, 615, 691, 736, or 849) when using a CRISPR-SaCas9 system. A portion where a cytidine deaminase is split is not particularly limited, as long as two split fragments can be reconstituted into a functional cytidine deaminase. A cytidine deaminase may be split at one location into an N-terminus side fragment and a C-terminus side fragment, or split at two or more locations and the three or more resulting fragments can be appropriately linked into two fragments. The three dimensional structure of a cytidine deaminase is known. Those skilled in the art can appropriately select the location of splitting based on said information.

As used herein, "compact cytidine deaminase" refers to a cytidine deaminase having reduced molecular weight compared to a wild-type cytidine deaminase by deleting some of the amino acid residues of the wild-type cytidine deaminase. Specific examples of such a compact cytidine deaminase include, in one embodiment, a cytidine deaminase consisting of:
(1) an amino acid sequence consisting of a region of amino acid residues at positions 30 to 150 in the amino acid sequence set forth in SEQ ID NO: 1;
(2) an amino acid sequence consisting of a region corresponding to the region of (1) which is an ortholog of a protein consisting of the amino acid sequence set forth in SEQ ID NO: 1;
(3) the amino acid sequence of (1) or (2) with one or several amino acid deletions, substitutions, insertions, and/or additions; or
(4) an amino acid sequence with 90% or greater similarity or identity with the amino acid sequence of (1) or (2).
However, the cytidine deaminase of (3) and (4) excludes wild-type cytidine deaminase and a fragment of said cytidine deaminase comprising at least the region of positions 28 to 161 of SEQ ID NO: 1 (region consisting of 134 amino acids). Hereinafter, the term "cytidine deaminase" can be simply used as a term encompassing both "compact cytidine deaminase" and "wild-type cytidine deaminase".

A cytidine deaminase consisting of the amino acid sequence set forth in SEQ ID NO: 1 is PmCDA1 (*Petromyzon marinus* cytosine deaminase 1) derived from a lamprey. Examples of orthologs of said PmCDA1 include AID (Activation-induced cytidine deaminase; AICDA) derived from a mammal (e.g., human, pig, cow, horse, monkey, etc.) For example, GenBank accession Nos. EF094822 and ABO15149 can be referred for the base sequence and amino acid sequence of cDNA of PmCDA1, and GenBank accession Nos. NM_020661 and NP_065712 can be referred for the base sequence and amino acid sequence of cDNA of human AID.

Regarding (3), more specific examples includes cytidine deaminases comprising (i) the amino acid sequence set forth in SEQ ID NO: 1 with deletions of 1 to 50, preferably 1 to 20, and more preferably 1 to several (5, 4, 3, or 2) amino acids, (ii) the amino acid sequence set forth in SEQ ID NO: 1 with additions of 1 to 50, preferably 1 to 20, and more preferably 1 to several (5, 4, 3, or 2) amino acids, (iii) the amino acid sequence set forth in SEQ ID NO: 1 with insertions of 1 to 50, preferably 1 to 20, and more preferably 1 to several (5, 4, 3, or 2) amino acids, (iv) the amino acid sequence set forth in SEQ ID NO: 1 with substitutions of 1 to 50, preferably 1 to 20, and more preferably 1 to several (5, 4, 3, or 2) amino acids with other amino acids, or (v) an amino acid sequence of a combination thereof.

The Examples presented below have demonstrated an effect of improving the efficiency of alteration by a cytidine deaminase presumed to be caused by stabilization of a protein due to substituting a hydrophobic amino acid residue exposed to the outside in the cytidine deaminase with a hydrophilic amino acid residue. Thus, it is preferable to substitute a hydrophobic amino acid residue exposed to the outside of a cytidine deaminase described above with a hydrophilic amino acid residue. Examples of such a hydrophobic amino acid residue exposed to the outside, with PmCDA1 as an example, include position 34 (Y), position 36 (L), position 50 (W), position 52 (Y), position 54 (V), position 74 (Y), position 94 (W), position 105 (L), position 122 (W), position 126 (L), position 136 (I), position 139 (W), and 150 (V), and amino acid residues at a position selected from positions corresponding to said amino acid residues in a cytidine deaminase derived from an animal other than lampreys (alphabet in the parenthesis indicates an amino acid residue). In particular, an amino acid residue at a position selected from the group consisting of positions 122, 126, and 139 is preferable. Examples of a hydrophilic amino acid residue include arginine, asparagine, aspartic acid, glutamic acid, glutamine, lysine, serine, and threonine.

The amino acid sequence of cytidine deaminase is highly conserved among vertebrates. The amino acid sequence of a cytidine deaminase derived from a desired animal can be aligned with the amino acid sequence of PmCDA1 to identify a corresponding site subjected to deletion or a corresponding site of mutation. If a corresponding amino acid residue is a hydrophilic amino acid, the amino acid residue does not need to be substituted, but may be substituted with another hydrophilic amino acid residue. For example for human AID, the amino acid corresponding to S30 of PmCDA1 is the threonine in position 27, the amino acid corresponding to V150 of PmCDA1 is the isoleucine in position 138, the amino acid corresponding to W122 of PmCDA1 is the phenylalanine in position 109, the amino acid corresponding to L126 of PmCDA1 is the leucine in position 113, and the amino acid corresponding to W139 of PmCDA1 is the arginine in position 127. While these amino acid residues can be substituted with any hydrophilic amino acid residue described above, it is preferable, with PmCDA1 as an example, to substitute W122 (F109 of human AID) with a glutamic acid residue or glutamic acid residue, L126 (L113 of human AID) with an asparagine residue, and/or W139 (R127 of human AID) with an arginine residue or glutamine residue in a preferred embodiment. Meanwhile, a substitution of a specific amino acid residue such as those forming a loop between β sheets that is present in a catalytic domain of deaminase activity (region of positions 66 to 100 of PmCDA1 or region of positions 56 to 90 of human AID) is not preferable (in other words, such amino acid residues are preferably conserved). Examples of such an amino acid residue that is preferably not substituted, with PmCDA1 as an example, include F49 (D45 of human AID), I65 (C55 of human AID), Y78 (W68 of human AID), Y91 (F81 of human AID), L112 (L98 of human AID), Y128 (F115 of human AID), and a region of positions 145 to 150 (region of positions 133 to 138 of human AID) forming the loop described above, and amino acid residues corresponding to said amino acid residues in a cytidine deaminase derived from an animal other than sea lamprey.

Two of more hydrophobic amino acid residues exposed to the outside of a cytidine deaminase described above may be substituted with a hydrophilic amino acid residue. Examples thereof include, with PmCDA1 as an example, two or more of amino acid residues at positions selected from the group consisting of positions 122, 126, and 139 (e.g., positions 122 and 139), etc. Specific examples include substitutions including combinations of mutations such as W122E/W139R, W122E/W139Q, W122Q/W139R, and W122Q/W139Q.

As used herein, "similarity" of amino acid sequences refers to the ratio (%) of: the same and similar amino acid residues, to all overlapping amino acid residues in the optimal alignment when two amino acid sequences are aligned by using a known mathematical algorithm in the art (preferably, the algorithm can consider introduction of a gap to one or both sequences for optimal alignment) . "Similar amino acid" refers to an amino acid which is similar in the physicochemical property. Examples thereof include amino acids classified into the same group such as aromatic amino acids (Phe, Trp, Tyr), aliphatic amino acids (Ala, Leu, Ile, Val), polar amino acids (Gln, Asn), basic amino acids (Lys, Arg, His), acidic amino acids (Glu, Asp), amino acids with a hydroxyl group (Ser, Thr), and amino acids with a small side chain (Gly, Ala, Ser, Thr, Met). It is expected that a substitution with such a similar amino acid does not result in a change in the phenotype of a protein (i.e., conservative amino acid substitution). Specific examples of a conservative amino acid substitution are well known in the art and are described in various documents (see, for example, Bowie et al., Science, 247: 1306-1310 (1990)). Similarity or identity of amino acid sequences herein can be calculated under the following conditions (expected value = 10; allow gap; matrix = BLOSUM62; filtering = OFF) by using a homology calculation algorithm NCBI BLAST (National Center for Biotechnology Information Basic Local Alignment Search Tool).

As used herein, "alteration" of a double stranded DNA refers to conversion of a nucleotide (e.g., dC) on a DNA strand into another nucleotide (e.g., dT, dA, or dG), deletion thereof, or insertion of a nucleotide or nucleotide sequence between nucleotides on a DNA strand. In this regard, an altered double stranded DNA is not particularly limited, as long as it is a double stranded DNA that is present in a host cell, but is preferably a genomic DNA (e.g., chromosomal DNA, mitochondrial DNA, chloroplastic DNA, etc.). Thus, alteration of a targeted site of a double stranded DNA refers to conversion of one or more nucleotides of the targeted site with one or more other nucleotides, deletion thereof, or insertion of one or more nucleotides into the targeted site. "Targeted site" of a double stranded DNA refers to all or part of a "target nucleotide sequence", which is specifically recognized and bound by a nucleic acid sequence recognition module, or the vicinity (one of both of 5' upstream and 3' downstream) of the target nucleotide sequence. The range thereof can be appropriately adjusted between one base and hundreds of bases long in accordance with the objective.

As used herein, "nucleic acid sequence recognition module" refers to a molecule or molecule complex having the ability to specifically recognize and bind to a specific nucleotide sequence (e.g., target nucleotide sequence) on a DNA strand. A nucleic acid sequence recognition module binds to a target nucleotide sequence to enable a cytidine deaminase linked to the module to specifically act on a targeted site of a double stranded DNA.

The complex of the present disclosure (also referred to as a "nucleic acid altering enzyme complex") refers to a molecule complex prepared from linking the nucleic acid sequence recognition module described above with a cytidine deaminase, having deamination activity with a specific nucleotide sequence recognizing capability imparted. In this regard, "complex" encompasses not only those comprised of a plurality of molecules, but also those having a nucleic acid sequence recognition module and a cytidine deaminase within a single molecule much like a fusion protein. Accordingly, a complex may be comprised of a guide RNA, a Cas effector protein (also referred to as a Cas protein or Cas nuclease), and a cytidine deaminase, or a complex may be comprised of a guide RNA and a fusion protein of a Cas effector protein and a cytidine deaminase, when using a CRISPR-Cas system as a nucleic acid sequence recognition module.

As described above, the complex of the present disclosure can efficiently alter a double stranded DNA without comprising a base excision/repair inhibitor. However, the Examples presented below have demonstrated use of a compact cytidine deaminase can reduce an off-target effect more significantly than from using a wild-type cytidine deaminase, even if a uracil-DNA glycosylase inhibitor is used concomitantly. It is presumed that such suppression of an off-target effect is due to removal of a domain of a cytidine deaminase with affinity to a DNA which is not desirable. Thus, the complex of the present disclosure may be further linked to a uracil-DNA glycosylase inhibitor.

Examples of the uracil-DNA glycosylase inhibitor used in the present disclosure include, but are not limited to, *Bacillus subtilis* bacteriophage PBS1 -derived uracil-DNA glycosylase inhibitors (UGI) and *Bacillus subtilis* bacteriophage PBS2 -derived uracil-DNA glycosylase inhibitors (UGI) (Wang, Z., and Mosbaugh, D. W. (1988) J. Bacteriol. 170, 1082-1091). In particular, PBS2 derived UGIs are known to have an effect of minimizing mutations other than C to T, cleavages, and recombinations on a DNA. Thus, use of a PBS2 derived UGI is suitable.

The target nucleotide sequence in a double stranded DNA recognized by a nucleic acid sequence recognition module is not particularly limited, as long as the module can specifically bind thereto. The target nucleotide sequence may be any sequence in a double stranded DNA. A target nucleotide sequence only needs to be sufficiently long for a nucleic acid sequence recognition module to specifically bind thereto. If, for example, a mutation is introduced into a specific site in a genomic DNA of a mammal, the length is 12 nucleotides or longer, preferably 15 nucleotides or longer, and more preferably 17 nucleotides or longer in accordance with the genome size. While the upper limit of length is not particularly limited, the length is preferably 25 nucleotides or less.

Examples of such a nucleic acid sequence recognition module that can be used include, but are not limited to, DNA binding domains of a protein that can specifically bind to a DNA such as restriction enzymes, transcription factors, and RNA polymerases in addition to CRISPR-Cas system wherein at least one DNA cleaving capability of a Cas protein is inactivated (hereinafter, also referred to as the "CRISPR-mutant Cas system"), a zinc finger motif, a TAL effector, a PPR motif, etc., as well as fragments that do not have a DNA double strand cleaving capability. Preferred examples include a CRISPR-mutant Cas system, a zinc finger motif, a TAL effector, a PPR motif, etc.

A zinc finger motif is 3 to 6 different Cys2His2 zinc finger units that are linked (one finger recognizes about 3 bases), which can recognized a 9 to 18 base target nucleotide sequence. A zinc finger motif can be prepared by a known method such as: a Modular assembly approach (Nat Biotechnol (2002) 20: 135-141), OPEN (Mol Cell (2008) 31: 294-301), CoDA (NatMethods (2011) 8: 67-69), or E. coli one-hybrid system (Nat Biotechnol (2008) 26: 695-701). Japanese Patent No. 4968498 can be referred for detailed preparation of zinc finger motifs.

A TAL effector has a repeat structure of modules having about 34 amino acids as a unit. The binding stability and base specificity are determined by the 12^{th} and 13^{th} amino acid residues (known as RVD) of one module. Since each module is highly independent, a TAL effector specific to a target nucleotide sequence can be prepared simply by connecting modules. Preparation methods of TAL effectors utilizing open resources (REAL (CurrProtoc Mol Biol (2012) Chapter 12: Unit 12.15), FLASH (Nat Biotechnol (2012) 30: 460-465), Golden Gate (Nucleic Acids Res (2011) 39: e82), etc.) are established. A TAL effector for a target nucleotide sequence can be designed relatively easily. Japanese National Phase PCT Laid-open Publication No. 2013-513389 can be referred for the detailed preparation of TAL effectors.

A PPR motif consists of 35 amino acids, and is configured to recognize a specific nucleotide sequence with consecutive PPR motifs that recognize a single nucleic acid base. A target base is recognized only with the 1^{st}, 4^{th}, and ii(-2)^{th} amino acids of each motif. Since there is no dependence on the motif configuration or interference from motifs on both sides, a PPR protein specific to a target nucleotide sequence can be prepared simply by connecting PPR motifs, just like TAL effectors. Japanese Laid-Open Publication No. 2013-128413 can be referred for detailed preparation of PPR motifs.

When using fragments of a restriction enzyme, transcription factor, RNA polymerase, etc., DNA binding domains of these proteins are well known, so that a fragment that comprises such a domain but does not have DNA double strand cleavage capability can be readily designed and constructed.

One of the nucleic acid sequence recognition modules described above can be provided as a fusion protein with a cytidine deaminase, or a protein binding domain such as a SH3 domain, PDZ domain, GK domain, or GB domain and a binding partner thereof may be fused to a nucleic acid sequence recognition module and a cytidine deaminase, respectively and provided as a protein complex via an interaction between the domain and the binding partner thereof. Alternatively, each of a nucleic acid sequence recognition module and a cytidine deaminase can be fused to an intein and linked by ligation after synthesizing each protein.

For a zinc finger motif there is not a highly efficient way of preparing a zinc finger that specifically binds to a target nucleotide sequence, and sorting out zinc fingers with high binding specificity is complex. Thus, it is not easy to prepare a large number of zinc finger motifs that actually function. While TAL effectors and PPR motifs have a higher degree of freedom for target nucleic acid sequence recognition compared to zinc finger motifs, a large protein needs to be designed and constructed for each target nucleotide sequence, such that there is a problem in terms of efficiency.

In contrast, a CRISPR-Cas system recognizes a sequence of a double stranded DNA of interest with a guide RNA complementary to a target nucleotide sequence. Thus, any sequence can be targeted simply by synthesizing an oligo DNA that can specifically form a hybrid with a target nucleotide sequence.

Thus, in a more preferred embodiment of the present disclosure, a CRISPR-mutant Cas system wherein one or both DNA cleaving capability of Cas is inactivated is used as a nucleic acid sequence recognition module.

A CRISPR-mutant Cas system is provided as a complex of a CRISPR-RNA (crRNA) comprising a sequence that is complementary to a target nucleotide sequence, optionally a trans-activating RNA (tracrRNA) required for recruiting a mutant Cas effector protein (if a tracrRNA is required, can be provided as a chimeric RNA prepared with a crRNA), and a mutant Cas effector protein. RNA molecules consisting of a crRNA alone or a chimeric RNA of a crRNA and tracrRNA, constituting a nucleic acid sequence recognition module in combination with a mutant Cas effector protein, are collectively referred to as a "guide RNA". As used herein, "targeted strand" refers to the strand that forms a hybrid with a crRNA, and the opposite strand that is in a single stranded state from hybrid formation of a targeted strand and a crRNA is referred to as a "non-targeted strand". When a target nucleotide sequence is expressed by one of the strands (e.g., when denoting a PAM sequence, when representing the positional relationship between a target nucleotide sequence and PAM, etc.), this is represented with the sequence of a non-targeted strand.

A Cas effector protein used in the present disclosure is not particularly limited, as long as the Cas effector protein can from a complex with a guide RNA and recognize and bind to a target nucleotide sequence in a gene of interest and protospacer adjacent motif (PAM) adjacent thereto, but is preferably Cas9 or Cpf1. Examples of Cas9 include, but are not limited to: *Streptococcus pyogenes* derived Cas9 (SpCas9; PAM sequence NGG (N is A, G, T, or C; the same applies hereinafter)), *Streptococcus thermophiles* derived Cas9 (StCas9; PAM sequence NNAGAAW), *Neisseria meningitidis* derived Cas9 (NmCas9; PAM sequence NNNNGATT), *Staphylococcus aureus* derived Cas9 (SaCas9; PAM sequence: NNGRR(T)), and *Campylobacter jejuni* derived Cas9 (CjCas9; PAM sequence NNNVRYM (V indicates A, G, or C; R indicates A or G; Y indicates T or C; M indicates A or C). From the viewpoint of restriction due to PAM, SpCas9 is preferable (which is substantially two bases, and almost anywhere on the genome can be theoretically targeted). From the viewpoint of size, Cas9 is preferably SaCas9 or CjCas9. Examples of Cpf1 include, but are not limited to, *Francisella novicida* derived Cpf1 (FnCpf1; PAM sequence NTT), *Acidaminococcus sp.* derived Cpf1 (AsCpf1; PAM sequence NTTT), *Lachnospiraceae* bacterium derived Cpf1 (LbCpf1; PAM sequence NTTT), etc. As a mutant Cas effector protein used in the present disclosure, those that have inactivated cleavage capability of both strands of a double stranded DNA of a Cas effector protein, and those that have only a cleavage capability of one strand and have nickase activity can be both used. For example for SpCas9, a D10A mutant which lacks an ability to cleave the opposite stand (i.e., "non-targeted strand") of the strand forming a complementary strand with a guide RNA (e.g., "targeted strand") (thus having nickase activity on a strand forming a complementary strand with a guide RNA) with the Asp residue in position 10 converted to an Ala residue, an H840A mutant that lacks an ability to cleave a strand forming a complementary strand with a guide RNA (thus having nickase activity on the opposite strand of a strand forming a complementary strand with a guide RNA) with the His residue in position 840 converted to an Ala residue, or a double mutant thereof (dCas9) can be used. For SaCas9, a mutant with the Asp residue in position 10 converted to an Ala residue and/or the Asp residue in position 556, His residue in position 557, and/or Asn residue in position 580 converted to an Ala residue can be prepared. For CjCas9, a mutant with the Asp residue in position 8 converted to an Ala residue and/or the His residue in position 559 converted to an Ala residue can also be used. For FnCpf1, a mutant lacking an ability to cleave both strands with the Asp residue in position 917 converted to an Ala residue (D917A) or the Glu residue in position 1006 converted to an Ala residue (E1006A) can be used. Other mutant Cas effector proteins can also be similarly used, as long as it lacks an ability to cleave at least one of the strands of a double stranded DNA.

A Cas effector protein may comprise an additional deletion or mutation besides the mutations described above. For example, mutant Cas effector proteins having a PAM recognition sequence that is different from a wild-type protein are known. Examples of such proteins include a mutant of SpCas9 of E108G/S217A/A262T/S409I/E480K/E543D/M694I/E1219V (xCas9 3.6), a mutant of SpCas9 of A262T/R324L/S409I/E480K/E694D/M694I/E1219V (xCas9 3.7) (PAM sequence: NG, GAA, and GAT) (Hu JH, et al., Nature., 556 (7699) : 57-63 (2018)), a mutant of SpCas9 of R1335V/L1111R/D1135V/G1218R/E1219F/A1322R/T1337R (SpCas9-NG) (PAM sequence: NGN) (Nishimasu H, et al., Science., 361 (6408): 1259-1262 (2018)), a mutant of SpCas9 of A262T/R324L/S409I/E480K/E543D/M694I/L1111R/D1135V/G1218R/E1 219F/A1322R/R1335V/T1337R (xCas9-NG) prepared by combining these mutations (Legut M, et al., Cell Rep, 30(9): 2859-2868 (2020)), a mutant of SpCas9 of D1135L/51136W/G1218K/E1219Q/R1335Q/T1337R (SpG) (PAM sequence: NGN), a mutant of SpCas9 of D1135L/51136W/G1218K/E1219Q/R1335Q/T1337R/L1111R/A1322R/A61 R/N1317R/R1333P (SpG) (PAM sequence: NRN and NYN) (Walton RT, et al., Science, 368 (6488): 290-296 (2020)), a mutant of SpCas9 of D1135V/R1335Q/T1337R/ (SpCas9-VQR) (PAM sequence: NGA), a mutant of SpCas9 of D1135V/G1218R/R1335E/T1337R (SpCas9-VERE) (PAM sequence: NGCG), a mutant of SaCas9 of E782K/N968K/R1015H (SaCas9-KKH) (PAM sequence: NNRRRT), SpCas9-NRRH, SpCas9-NRTH, and SpCas9-NRCH (PAM sequence: NRRH, NRCH, and NRTH, respectively) (Miller SM, et al., Nat Biotechnol. 38(4) :471-481 (2020), etc.

### 2. Nucleic acid encoding a complex for altering a double stranded DNA

The complex of the present disclosure comprising a complex of a nucleic acid sequence recognition module bound with a deaminase (e.g., cytidine deaminase) may be contacted with a double stranded DNA by introducing a nucleic acid encoding the complex (hereinafter, also referred to as the "nucleic acid of the present disclosure") into a cell having a double stranded DNA of interest. The complex of the present disclosure and each constituent molecule of the complex can also be manufactured by a molecular biological methodology using the nucleic acid of the present disclosure. Thus, a nucleic acid sequence recognition module and a cytidine deaminase may be prepared as a nucleic acid encoding a fusion protein thereof, or as a nucleic acid encoding each of them in a form in which a complex can be formed within a host cell after translation into a protein by utilizing a binding domain, intein, etc. In this regard, a nucleic acid may be a DNA or an RNA. A DNA is preferably a double stranded DNA and is provided in a form of an expression vector placed under the control of a functional promoter in a host cell or in a form of an expression vector comprising the DNA. An RNA is preferably a single stranded RNA.

As used herein, "encoding a complex" encompassed both encoding each molecule constituting the complex and encoding a fusion protein having two or more constituent molecules within a single molecule.

A DNA encoding a nucleic acid sequence recognition module such as a zinc finger motif, TAL effector, or PPR motif can be obtained by any method described above for each module. A DNA encoding a sequence recognition module such as a restriction enzyme, transcription factor, or RNA polymerase can be cloned by, for example, synthesizing an oligo DNA primer to cover a region encoding a desired portion of the protein (portion comprising a DNA binding domain) based on cDNA sequence information thereof and amplifying it by RT-PCR using a total RNA or mRNA fraction prepared with a cell producing said protein as a template.

A DNA encoding a cytidine deaminase can also be cloned in the same manner by synthesizing an oligo DNA primer to achieve a deletion of a desired amino acid residue based on cDNA sequence information of the used cytidine deaminase and amplifying it by RT-PCR using a total RNA or mRNA fraction prepared witha cell producing said cytidine deaminase as a template. For example, a DNA encoding compact PmCDA1 of lampreys can be cloned by designing a suitable primer for a suitable region of CDS based on the cDNA sequence (accession No. EF094822) registered in the NCBI database and using RT-PCR from an mRNA derived from lampreys. A DNA encoding human AID can also be cloned in the same manner based on the cDNA sequence (accession No. AB040431) registered in the NCBI database. When using a donor DNA for alteration of a targeted site, the donor DNA can also be cloned in the same manner described above, based on sequence information of the site, etc.

A DNA encoding a fusion protein can be prepared by ligating a DNA encoding a nucleic acid sequence recognition module to a cloned DNA directly or a cloned DNA digested with a restriction enzyme or added with a suitable linker and/or nuclear localization signal (each organelle localization signal if a double stranded DNA of interest is a mitochondrial or chloroplastic DNA) when desired. Alternatively, a DNA encoding a nucleic acid sequence recognition module and a DNA encoding a cytidine deaminase may each be fused to a binding domain or its binding partner encoding DNA, or both DNAs may be fused to a DNA encoding a separation intein, so that a complex can be formed after a nucleic acid sequence recognition conversion module and a cytidine deaminase are translated in a host cell. In such a case, a linker and/or nuclear localization signal can be linked at a suitable position of one or both DNAs when desired. When a donor DNA is used for alteration of a targeted site, the donor DNA may be prepared as a single DNA or provided as a single DNA comprising a nucleic acid encoding a nucleic acid sequence recognition module and/or a cytidine deaminase.

For a DNA encoding a nucleic acid sequence recognition module, a DNA encoding a cytidine deaminase, and a donor DNA, a DNA encoding the full length can be constructed by chemically synthesizing a DNA strand or connecting a synthesized partial overlapped oligo DNA short strand by utilizing PCR or Gibson assembly. Examples of methods other than chemically synthesizing a DNA strand, when a donor DNA is a single stranded nucleic acid, include preparation thereof by digesting a plasmid DNA comprising said DNA with a restriction enzyme to prepare a single strand, synthesizing an RNA through an RNA polymerase, then synthesizing a cDNA with a reverse transcriptase, and decomposing an RNA strand with RNaseH. Alternatively, such a DNA can be prepared by digesting a plasmid comprising a donor DNA with a nickase restriction enzyme and separating/purifying the same through electrophoresis. Construction of a full length DNA by chemical synthesis or combination with PCR or Gibson assembly is advantageous in that a codon, which is used, can be designed over the full length of CDS in accordance with a host to which the DNA is introduced. Upon expression of a heterologous DNA, an increase in the level of protein expression can be expected by converting the DNA sequence into a codon with high usage frequency in a host organism. For example, the codon usage database published on the website of Kazusa DNA Research Institute (http://www.kazusa.or.jp/codon/index.html) can be used for data on codon usage frequency in a host which is used. Alternatively, areference describing the codon usage frequency in each host can be referenced. The obtained data and DNA sequence to be introduced may be referenced to convert a codon with a low usage frequency in a host among codons used in said DNA sequence with a codon with a high usage frequency encoding the same amino acid.

An expression vector comprising a nucleic acid encoding a nucleic acid sequence recognition module and/or deaminase (e.g., cytidine deaminase) can be manufactured, for example, by linking said DNA downstream of a promoter in a suitable expression vector.

As an expression vector, an *E. coli* derived plasmid (e.g., pBR322, pBR325, pUC12, or pUC13); *Bacillus subtilis* derived plasmid (e.g., pUB110, pTP5, or pC194); yeast derived plasmid (e.g., pSH19 or pSH15); insect cell expressed plasmid (e.g.: pFast-Bac); animal cell expressed plasmid (e.g.: pA1-11, pXT1, pRc/CMV, pRc/RSV, or pcDNAI/Neo); bacteriophage such as A phage; insect viral vector such as *Baculoviridae* viral vector (e.g.: BmNPV or AcNPV); animal viral vector such as a retrovirus, vaccinia virus, adenovirus, or adeno-associated virus (AAV) vector; etc. is used. If use in gene therapy is considered, an AAV vector is suitably used from the viewpoint of long-term expression in an introduced gene and safety in view of being non-pathogenic virus derived.

Various deaminases can be used in the present disclosure. For example, a cytidine deaminase has a reduced molecular weight compared to a wild-type cytidine deaminase. Thus, a nucleic acid encoding a nucleic acid sequence recognition module and a nucleic acid encoding a cytidine deaminase can be inserted into a single AAV vector by using a nucleic acid sequence recognition module with a low molecular weight (e.g., SaCas9, CjCas9, etc.) as needed. Alternatively, the molecular weight can be reduced by deleting a portion of a nucleic acid base module (e.g., deleting positions 1024 to 1054 of SpCas9). Specifically, when using a CRISPR-Cas system as a nucleic acid sequence recognition module, a nucleic acid encoding a Cas effector protein, a nucleic acid encoding a guide RNA, and a nucleic acid encoding a cytidine deaminase can all be inserted into a single AAV vector. As used herein, "nucleic acid sequence recognition module" encompasses not only wild-type, but also variants thereof having a nucleic acid sequence recognition capability (e.g., variants of SpCas9 described above, etc.).

When using a viral vector as an expression vector, it is preferable to use a vector derived from a serotype suitable for infection of tissue or organ of interest. Preferred examples of an AAV vector used include AAV1, 2, 3, 4, 5, 7, 8, 9, or 10 based vectors when targeting the central nervous system or retina, AAV 1, 3, 4, 6, or 9 based vectors when targeting the heart, AAV1, 5, 6, 9, or 10 based vectors when targeting the lung, AAV 2, 3, 6, 7, 8, or 9 based vectors when targeting the liver, and AAV1, 2, 6, 7, 8, or 9 based vectors when targeting the skeletal muscle. For cancer therapy, it is preferable to use AAV2. For example, WO 2005/033321 A2, etc. can be referred for AAV serotypes.

A promoter may be any promoter, as long as it is compatible and suitable for a host used in expression of a gene. Since conventional methods involving DSB may significantly reduce the survival rate of a host cell due to toxicity, it is desirable to increase the cell count until start of induction by using an induction promoter. Meanwhile, sufficient cell growth is achieved even if the complex of the present disclosure is expressed. Thus, constituent promotors can also be used without restriction.

If a host is, for example, an animal cell, SRα promoter, SV40 promoter, LTR promoter, CMV (cytomegalovirus) promoter, RSV (Rous sarcoma virus) promoter, MoMuLV (moloney murine leukemia virus) LTR, HSV-TK (herpes simplex virus thymidine kinase) promoter, etc. areused. In particular, CMV promoter, SRα promoter, etc. are preferable.

When a host is *E. coli,* trp promoter, lac promoter, recA promoter, λP_{L} promoter, lpp promoter, T7 promoter, etc. are preferable.

When a host is a *Bacillus* bacteria, SPO1 promoter, SPO2 promoter, penP promoter, etc. are preferable.

When a host is a yeast, Gal1/10 promoter, PHO5 promoter, PGK promoter, GAP promoter, ADH promoter, etc. are preferable.

When a host is an insect cell, polyhedrin promoter, P10 promoter, etc. are preferable.

When a host is a plant cell, CaMV35S promoter, CaMV19S promoter, NOS promoter, etc. are preferable.

In addition to those described above, an expression vector comprising an enhancer, splicing signal, terminator, polyadenylation signal, drug resistant gene, auxotrophic complementary gene, other selection markers, replication point, etc. can also be used as desired.

An RNA encoding a nucleic acid sequence recognition module and/or cytidine deaminase can be prepared by, for example, transcription to an mRNA in a known in vitro transcription system using the DNA encoding a nucleic acid sequence recognition module and/or cytidine deaminase described above as a template.

A DNA encoding a guide RNA can be chemically synthesized by using a DNA/RNA synthesizer after designing an oligo DNA sequence from linking a coding sequence of a crRNA sequence (e.g., when FnCpf1 is recruited as a Cas effector protein, a crRNA comprising SEQ ID NO: 2: AAUUUCUACUGUUGUAGAU on the 5' side of a targeting sequence can be used, and the underlined sequences form a base pair with each other to have a stem loop structure), comprising a nucleotide sequence complementary to a target nucleotide sequence (also referred to as a "targeting sequence" herein), or crRNA coding sequence and optionally a known tracrRNA coding sequence (e.g., gttttagagctagaaatagcaagttaaaataaggctagtccgttatcaacttgaaaaag tggcaccgagtcggtgcttttttt; SEQ ID NO: 6 (for SpCas9), gttttagtactctggaaacagaatctactaaaacaaggcaaaatgccgtgtttatctcg tcaacttgttggcgagattttttt; SEQ ID NO: 7 (for SaCas9), etc., as a tracrRNA coding sequence when Cas9 is recruited as a Cas effector protein).

The length of a targeting sequence is not particularly limited, as long as the targeting sequence can specifically bind to a target nucleotide sequence. The length is, for example, 15 to 30 nucleotides, and preferably 18 to 25 nucleotides.

A targeting sequence can be designed by listing up 20mer sequences adjacent to PAM (e.g., NGG for SpCas9) on the 3' side in a CDS sequence of a gene of interest and selecting a sequence that would result in a change in an amino acid in a protein encoded by the gene of interest when C is converted to T within 7 nucleotides from the 5' end to the direction of 3' by using a published guide RNA design website (CRISPR DesignTool, CRISPRdirect, etc.) when using Cas9 as a Cas effector protein. A sequence can also be appropriately selected when using a length of targeting sequence other than 20mer. Among these candidates, a candidate sequence with few off-target sites in a host genome of interest can be used as a targeting sequence. If the guide RNA designing software used lacks a function for searching for an off-target site of a host genome, an off-target site can be search by, for example, performing Blast search on a host genome for 8 to 12 nucleotides on the 3' side of a candidate sequence (seed sequence with high capability of distinguishing a target nucleotide sequence).

A DNA encoding a guide RNA can also be inserted into an expression vector similar to those described above. As a promoter, it is preferable to use a pol III promoter (e.g., SNR6, SNR52, SCR1, RPR1, U3, U6, H1 promoter, etc.) and a terminator (e.g., polyT sequence (T₆ sequence, etc.)).

### 3. Method of altering a targeted site of a double stranded DNA

Another embodiment provides a method of altering a targeted site of a double stranded DNA, comprising contacting the complex of the present disclosure with the double stranded DNA of a host cell (hereinafter, also referred to as the "alteration method of the present disclosure"). The complex of the present disclosure including a complex of a nucleic acid sequence recognition module bound with a cytidine deaminase (including a fusion protein) and double stranded DNA may be contacted as an enzymatic reaction in an acellular system, but it is desired to introduce the complex, nucleic acid, or vector of the present disclosure described in section 1 or 2 into a host and culture the host cell, which is in accordance with the primary object of the present disclosure.

The inventors have previously demonstrated that introduction of a complex of a nucleic acid sequence recognition module and a cytidine deaminase into cell results in not only conversion of a nucleotide at a target site, but also deletion or insertion of one or more nucleotides (International Publication No. WO 2015/133554). Thus, alteration of a targeted site may be not only conversion of one or more nucleotides at a targeted site into one or more other nucleotides, but also one or more nucleotide deletions or insertion of one or more nucleotides into a targeted site. Furthermore, the inventors have previously demonstrated that further introduction of an exogenous donor DNA into a host can, by a homologous recombination mechanism, substitute a target site in a double stranded DNA with an insertion sequence contained in the exogenous donor DNA or insert the insertion sequence into the target site (International Publication No. WO 2019/189147). Accordingly, the alteration method of the present disclosure may comprise a step of introducing a donor DNA into a cell.

A complex of a nucleic acid sequence recognition module and a deaminase (e.g., cytidine deaminase) can be expressed in a cell by introducing a nucleic acid encoding the nucleic acid sequence recognition module and/or deaminase (e.g., cytidine deaminase) or an expression vector comprising said nucleic acid into a host cell and culturing the host cell. Since the alteration method of the present disclosure does not involve DNA double-strand break (DSB), genome editing with low toxicity is possible. Such a method can be applied in wide-ranging biological materials. Thus, a cell introduced with a nucleic acid encoding a nucleic acid sequence recognition module and/or cytidine deaminase can encompass cells of any biological species, from cells of bacteria such as *E. coli* which are prokaryotes and microorganisms such as yeasts which are lower eukaryotes to cells of higher eukaryotes such as vertebrates including mammals such as humans, insects, and plants.

Examples of a host that can be used include *Escherichia* bacteria, *Bacillus* bacteria, yeasts, insect cells, insects, animal cells, etc.

Examples of *Escherichia* bacteria that can be used include *Escherichia coli* K12·DH1 [Proc. Natl. Acad. Sci. USA, 60, 160 (1968)], *Escherichia coli* JM103 [Nucleic Acids Research, 9, 309 (1981)], *Escherichia coli* JA221 [Journal of Molecular Biology, 120, 517 (1978)], *Escherichia coli* HB101 [Journal of Molecular Biology, 41, 459 (1969)], *Escherichia coli* C600 [Genetics, 39, 440 (1954)], etc.

Examples of *Bacillus* bacteria that can be used include *Bacillus subtilis* MI114 [Gene, 24, 255 (1983)], *Bacillus subtilis* 207-21 [Journal of Biochemistry, 95, 87 (1984)], etc.

Examples of yeasts that can be used include *Saccharomyces cerevisiae*) AH22, AH22R⁻, NA87-11A, DKD-5D, and 20B-12, *Schizosaccharomyces pombe* NCYC1913 and NCYC2036, *Pichia pastoris* KM71, etc.

Examples of insect cells that can be used, when a virus is AcNPV, include cabbage armyworm larva derived cell line (*Spodoptera frugiperda* cell; Sf cells), *Trichoplusiani midgut* derived MG1 cells, *Trichoplusia ni* egg derived High Five^{™} cells, *Mamestra brassicae* derived cells, *Estigmenaacrea* derived cells, etc. Examples of insect cells that can be used, when a virus is BmNPV, include silkworm derived cell lines (*Bombyx mori* N cells; BmN cells), etc. Examples of the Sf cells that can be used include Sf9 cells (ATCCCRL1711), Sf21 cells [In Vivo, 13, 213-217 (1977)], etc.

Examples of insects that can be used include silkworm larvae, vinegar flies, crickets, etc. [Nature, 315, 592 (1985)] .

Examples of animal cells that can be used include cell lines of monkey COS-7 cells, monkey Vero cells, Chinese hamster ovary (CHO) cells, dhfr gene knockout CHO cells, mouse L cells, mouse AtT-20 cells, mouse myeloma cells, rat GH3 cells, and human FL cells, iPS cells and ES cells of humans and other mammals and other pluripotent stem cells, and primary culture cells prepared from various tissues. Zebrafish embryos, xenopus oocytes, etc. can also be used.

Examples of plant cells that can be used include suspension cultured cells, calluses, protoplasts, leaf segments, root segments, etc. prepared from various plants (e.g., cereals such as rice, wheat, and corn, commercial crops such as tomato, cucumber, and eggplant, garden plants such as carnation and prairie gentian, experimental plants such as tobacco and *Arabidopsis thaliana,* etc.).

An expression vector can be introduced in accordance with a known method (e.g., lysozyme method, competent method, PEG method, CaCl₂ co-precipitation, electroporation, microinjection, particle gun method, lipofection, agrobacterium method, etc.) depending on the type of host. A donor DNA can also be introduced into cells by the same methods. When introducing an expression vector and a donor DNA as different molecules, the expression vector and donor DNA may be introduced simultaneously or at different timings.

*E. coli* can be transformed in accordance with the method described in, for example, Proc. Natl. Acad. Sci. USA, 69, 2110 (1972), Gene, 17, 107 (1982), etc.

A vector can be introduced into *Bacillus* bacteria in accordance with the method described in, for example, Molecular & General Genetics, 168, 111 (1979), etc.

A vector can be introduced into yeast in accordance with the method described in, for example, Methods in Enzymology, 194, 182-187 (1991), Proc. Natl. Acad. Sci. USA, 75, 1929 (1978), etc.

A vector can be introduced into insect cells or insects in accordance with the method described in, for example, Bio/Technology, 6, 47-55 (1988), etc.

A vector can be introduced into animal cells in accordance with the method described in, for example, Saibo Kogaku Bessatsu 8 [Cell Engineering Extra Issue 8] Shin Saibo Kogaku Jikken Purotokoru [New Cell Engineering Experimental Protocol, 263-267 (1995) (published by Shujunsha), Virology, 52, 456 (1973), etc.

Cells introduced with the nucleic acid of the present disclosure can be cultured in accordance with a known method depending on the type of host.

When, for example, *E*. *coli* or *Bacillus* bacteria is cultured, a liquid medium is preferable as the medium used in culture. A medium also preferably comprises a carbon source, nitrogen source, inorganic matter, etc. required for the growth of a transformant. Examples of the carbon source include glucose, dextrin, soluble starch, sucrose, etc. Examples of the nitrogen source include ammonium salts, nitrates, corn steep liquor, peptone, casein, meat extract, soybean meal, potato extract, and other inorganic and organic matters. Examples of inorganic matters include calcium chloride, sodium dihydrogen phosphate, magnesium chloride, etc. A yeast extract, vitamin, growth promoting agent, etc. may also be added to a medium. The pH of a medium is preferably about 5 to about 8.

Preferred examples of a medium when *E. coli* is cultured include an M9 medium comprising glucose and casamino acid [Journal of Experiments in Molecular Genetics, 431-433, Cold Spring Harbor Laboratory, New York 1972]. An agent such as 3β-indoleacrylic acid may be optionally added to a medium for efficient functioning of a promoter. *E*. *coli* is generally cultured at about 15 to about 43°C. Aeration and agitation may also be applied as needed.

*Bacillus* bacteria are generally cultured at about 30 to about 40°C. Aeration and agitation may also be applied as needed.

Examples of medium when yeast is cultured include Burkholder minimum medium [Proc. Natl. Acad. Sci. USA, 77, 4505 (1980)], SD medium comprising 0.5% casamino acid [Proc. Natl. Acad. Sci. USA, 81, 5330 (1984)], etc. The pH of a medium is preferably about 5 to about 8. Yeast is generally cultured at about 20°C to about 35°C. Aeration and agitation may also be applied as needed.

Examples of medium when insect cells or insects are cultured include Grace's Insect Medium [Nature, 195, 788 (1962)] with an appropriate addition of an additive such as inactivated 10% bovine serum. The pH of a medium is preferably about 6.2 to about 6.4. Insect cells or insects are generally cultured at about 27°C. Aeration and agitation may also be applied as needed.

Examples of medium when animal cells are cultured include a minimum essential medium (MEM) comprising about 5 to about 20% fetal bovine serum [Science, 122, 501 (1952)], Dulbecco's Modified Eagle Medium (DMEM) [Virology, 8, 396 (1959)], RPMI 1640 medium [The Journal of the American Medical Association, 199, 519 (1967)], 199 medium [Proceeding of the Society for the Biological Medicine, 73, 1 (1950)], etc. The pH of a medium is preferably about 6 to about 8. The cells are generally cultured at about 30°C to about 40°C. Aeration and agitation may also be applied as needed.

Examples of medium when plant cells are cultured include an MS medium, LS medium, B5 medium, etc. The pH of a medium is preferably about 5 to about 8. The cells are generally cultured at about 20°C to about 30°C. Aeration and agitation may also be applied as needed.

A complex of a nucleic acid sequence recognition module and a cytidine deaminase, i.e., the complex of the present disclosure, can be expressed in a cell in a manner described above.

An RNA encoding a nucleic acid sequence recognition module and/or compact deaminase can be introduced into a host cell by microinjection, lipofection, etc. An RNA can be introduced once or repeatedly for multiple times (e.g., 2 to 5 times) at a suitable interval.

As used herein, "donor DNA" refers to a DNA comprising an exogenous insertion sequence. A donor DNA generally comprises two types of sequences (hereinafter, also referred to as "homology arms") homologous to sequences of two regions at the upstream side and downstream side of a target site, which are adjacent to the target site (hereinafter, also referred to as "adjacent regions"). Each homology arm may be distinguished as the "5' homology arm" and "3' homology arm". A "target site" of a double stranded DNA refers to a region that would be substituted with an insertion sequence contained in thedonor DNA, or a site between nucleotides where the insertion sequence would be inserted. The target site does not include the aforementioned adjacent sequences. When a site other than the target nucleotide sequence and PAM sequence is the target site, the sequences may remain after alteration and result in deamination due to a cytidine deaminase. Thus, it is preferable to design a donor DNA so that these sequences are excluded, or to introduce a silent mutation into a target nucleotide sequence or PAM sequence on homology arms.

A sequence homologous to an adjacent region of a target site may be not only a completely identical sequence, but also a sequence with identity of preferably 80% or greater (e.g., 85% or greater, 90% or greater, 95% or greater, 96% or greater, 97% or greater, 98% or greater, or 99% or greater) to the completely identical sequence, as long as homologous recombination can occur within a cell.

An insertion sequence can optionally include a drug resistant gene (e.g., kanamycin resistant gene, ampicillin resistant gene, puromycin resistant gene, etc.), thymidine kinase gene, diphtheria toxin gene, other selection marker sequences, green fluorescent protein (GFP), red fluorescent protein, β glucuronidase (GUS), FLAG, other report gene sequences, etc. To enable these genes to be resected after completion of cell sorting, etc., an insertion sequence may have a LoxP sequence, FRT sequence, or transposon specific inverted terminal repeat (PiggyBac Terminal Repeat) in the front or back thereof. Examples of preferred transposon include piggyBac, which is a Lepidoptera insect derived transposon, etc. (Kaji, K. et al., Nature, 458: 771-775 (2009), Woltjen et al., Nature, 458: 766-770 (2009), WO 2010/012077). Alternatively, an expression vector comprising the aforementioned drug resistant gene may be co-introduced to perform transient (about several days) drug sorting, as described in Oji A et al., Sci Rep, 6: 31666 (2016), etc. Insertion of an insertion sequence into a target site or substitution with a target site can be confirmed by decoding the sequence, screening of a chromosomal DNA that is separated and extracted from a cell through Southern hybridization or PCR, etc. If the aforementioned drug resistant gene, etc. is present on a donor DNA, they can be confirmed by using the expression thereof as an indicator.

A donor DNA may be a linear (e.g., synthetic double stranded DNA) or cyclic (e.g., plasmid DNA), single stranded DNA (e.g., single stranded oligodeoxynucleotide (ssODN)) or double stranded DNA. A donor DNA can be appropriately designed depending on the base length of an insertion sequence, homologous recombination activity of a host cell, etc. For example, when the base length of an insertion sequence is 100 bases or less, ssODN or synthetic double stranded DNA is generally used. When longer, a synthetic double stranded DNA or plasmid DNA is generally used. The length of a donor DNA is not particularly limited. The length can be appropriately designed depending on the length of an insertion sequence, etc. The length of an insertion sequence is not particularly limited. The length can be appropriately designed in accordance with the objective, generally in the range of one base to 10s of thousands of bases (e.g., 100 bases or less for ssODN (e.g., 70 bases or less or 50 bases or less)). The length of each homology arm is also not particularly limited. When a donor DNA is ssODN, those with a length of 10 bases to 150 bases are generally used. When a donor DNA is a synthetic double stranded DNA, those with a length of 10 to 5000 bases are generally used. When a donor DNA is a plasmid DNA, those with a length of generally 100 bases to 5000 bases and preferably 500 bases to 1000 bases are used. Such donor DNAs can be designed by referring to a known document (e.g., Ochiai H, Int J Mol Sci, 16: 21128-21137 (2015), or Hockemeyer D et al., Nat Biotefchnol, 27: 851-857 (2009)).

The alteration method of the present disclosure can alter a targeted site by using a plurality of target nucleotide sequences at different positions. Thus in one embodiment of the present disclosure, two or more nucleic acid sequence recognition modules that specifically bind to different respective target nucleotide sequences can be used. In such a case, each of these nucleic acid sequence recognition modules forms a complex with a cytidine deaminase. In this regard, a common cytidine deaminase can be used. For example, when a CRISPR-mutant Cas system is used as a nucleic acid sequence recognition module, two or more crRNAs forming a complementary strand with different respective nucleotide sequences, or two or more chimeric RNAs of the two respective crRNAs and a tracrRNA can be prepared and used, as a guide RNA (crRNA, or chimera of crRNA-tracrRNA), by using a common complex of a mutated Cas effector protein and a cytidine deaminase. Meanwhile, when using a zinc finger motif, TAL effector, etc. as a nucleic acid sequence recognition module, a cytidine deaminase can be bound to, for example, each nucleic acid sequence recognition module specifically binding to a different target nucleotide.

To express the complex of the present disclosure within a host cell, an expression vector comprising a DNA encoding the complex is introduced into a host cell as described above. Meanwhile, in order to efficiently introduce a mutation, it is desirable that expression of a complex is maintained for acertain period or longer at a certain level or higher. From such a viewpoint, the expression vector is certainly incorporated into a host genome, but sustained expression of a complex increases the risk of off-target cleavage. Thus, it is preferable to quickly remove the DNA after alteration of a target site is successfully achieved. Examples of means for removing a DNA incorporated into a host genome include a method of using a Cre-loxP system or FLP-FRT system, method of using transposon, etc.

Alternatively, efficient editing of a host genome can be materialized while avoiding the risk of off-target cleavage by transiently expressing the complex of the present disclosure within a host cell only during a period required for a deamination reaction to occur at a desired time and for immobilizing an alteration of a targeted site. Those skilled in the art can appropriately determine a suitable expression induction period based on the culture conditions used, etc. An exemplary expression induction period can be 20 to 40 hours when, for example, budding yeasts are cultured in a 0.02% galactose induction medium.

Examples of means for transiently expressing the complex of the present disclosure for a desired period at a desired time include a method of preparing a construct (expression vector) comprising a nucleic acid encoding the complex in a form in which the expression period can be controlled, and introducing the construct into a host. Specific examples of "form in which the expression period can be controlled" include the nucleic acid of the present disclosure under control of an induction regulatory region. Examples of "induction regulatory region" include, but are not particularly limited to, an operon of a temperature sensitive (ts) mutant repressor and an operator controlled thereby. Examples of ts mutant repressors include, but are not limited to, ts mutants of a λ phage derived cI repressor. A λ phage derived cI repressor (ts) binds to an operator and suppresses gene expression downstream at 30°C or less (e.g., 28°C), but dissociates from an operator at a high temperature of 37°C or higher (e.g., 42°C) so that gene expression is induced. Hence, a host cell introduced with the nucleic acid of the present disclosure is generally cultured at 30°C or less, the temperature is raised to 37°C or higher at a suitable time, the cell is cultured for a certain period of time and subjected to a deamination reaction to introduce a mutation into a target gene. After this process, the temperature can be quickly returned to 30°C or less to minimize the period during which expression of the target gene is suppressed. Even if a gene that is essential for a host cell is targeted, the gene can be edited efficiently while suppressing side effects.

When a temperature sensitive mutation is utilized, insertion of the temperature sensitive mutation of a protein that is required for autonomous replication of a vector into a vector comprising a DNA encoding the complex of the present disclosure results in autonomous replication being disabled quickly after expression of the complex, and the vector naturally falling off with cell division. Examples of such a temperature sensitive mutant protein include, but are not limited to, a temperature sensitive mutant of Rep101 ori which is required for replication of pSC101 ori. At 30°C or less (e.g., 28°C), Rep101 ori (ts) acts on pSC101 ori to enable autonomous replication of a plasmid, but loses the function at 37°C or higher (e.g., 42°C), so that the plasmid would no longer be able to autonomously replicate. Thus, transient expression of the complex of the present disclosure and plasmid removal can be simultaneously performed by concurrent use with a cI repressor (ts) of λ phage described above.

Transient expression of the complex of the present disclosure can be materialized by introducing a DNA encoding the complex into a host cell under the control of an inducible promoter (e.g., lac promoter (induced by IPTG), cspA promoter (induced by cold shock), araBAD promoter (induced by arabinose), etc.), adding an inducer to a medium (or removing the inducer from a medium) at a suitable time to induce expression of the complex, culturing the cell for a certain period of time, and subjecting the cell to a nucleic acid alteration reaction to introduce a mutation into a target gene.

Hereinafter, the present disclosure is described through the Examples. However, the present disclosure is not limited to the Examples.

### [Examples]

### <Cell Line/Culture/Transformation/Expression Induction>

A budding yeast *Saccharomyces cerevisiae* BY4741 line (requiring leucine and uracil) was cultured in a Dropout composition matching the nutritional requirement of a standard YPDA medium or SD medium. The yeasts were cultured on an agar plate through static culture or in a liquid medium through shake culture between 25°C to 30°C. A lithium acetate method was used for transformation. Yeasts were sorted out in a suitable SD medium matching the nutritional requirement. To induce expression with galactose, the yeasts were precultured overnight in a suitable SD medium. The yeasts were then subcultured in an SR medium, where 2% glucose was replaced with 2% raffinose as a carbon source, and cultured overnight. The yeasts were subcultured in an SGal medium, where a carbon source was replaced with 0.2% galactose, and cultured for about 3 hours to 2 nights to induce expression.

The viable cell count and Can1 mutation rate were determined by appropriately diluting and applying a cell suspension to an SD plate medium and SD-Arg + 60 mg/l canavanin plate medium or SD + 300 mg/l canavanine plate medium and counting the number of colonies appearing after three days as the viable cell count. The mutation rate was calculated/evaluated by using the viable colony count on the SD plate as the total cell count and the viable colony count on the canavanine plate as the resistant mutant line count.

To investigate the off-target effect, a cell suspension was appropriately diluted and applied to an SD plate medium and SD + 100 mg/l S-aminoethyl-L-cysteine (Thialysine), and the number of colonies appearing after three days was counted as the viable cell count. The off-target mutation rate was calculated/evaluated by using the viable colony count on the SD plate as the total cell count and the viable colony count on the Thialysine plate as the resistant mutant line count.

### <Nucleic acid manipulation>

A DNA was processed/constructed by PCR, restriction enzyme treatment, ligation, Gibson assembly, or artificial chemical synthesis. For a plasmid, pRS415 for leucine selection was used as a yeast/E. *coli* shuttle vector, and pRS426 for uracil selection was used as the backbone. The plasmid was amplified with *E. coli* line XL-10 gold or DH5α, and introduced into yeasts by a lithium acetate method.

### <Construction of a construct>

Each domain was cleaved, replaced, and introduced with a mutation in accordance with the approach and plasmid described in Non Patent Literature 3. SaABEmax (#119814) was obtained from Addgene as a SaCas9 containing vector for expression in mammals and altered. Scp1 sequences and polyA signals were artificially chemically synthesized. The target sequences of gRNA of KN1086, KN1150, KN1025, and KN1149 are set forth in SEQ ID NO: 8 to 11, respectively. The full length sequences of pAL008, pAL022, V5679, pAL047, and pAL050 are set forth in SEQ ID NO: 12 to 16, respectively, as representatives of each construct. Vector 1251 used as a control in the following Examples comprises a sequence encoding conventional dCas9-dSH3-CDA (free of UGI), and vector 1252 comprises a sequence encoding conventional nCas9-dSH3-CDA (free of UGI) (dSH3 is a linker). Further, pAL008 comprises a sequence encoding CDA-nCas9 and is fused to CDA without a linker to the N-terminus side of Cas9.

### <Three-dimensional structure analysis>

The three dimensional structure of AID (id: 5W1C) was obtained from MMDB of NCBI and analyzed on a software (Cn3D). Alignment was performed with ClustalW.

### <Transfection and induction of mutation into HEK293 cells)

Human embryonic kidney cells (HEK293T cells) were used. The cells were cultured under the conditions of 37°C and 5% CO₂ by using a DME-glutamax medium (ThermoFisher Scientific, USA) added with 100 µg/mL penicillin-streptomycin (Life Technologies, Carlsbad, CA, USA) and 10% fetal bovine serum (FBS) (Biosera, Nuaille, France). The cells were retrieved using 5% trypsin. HEK293T cells preserved in a deep freezer were thawed in a 37°C water bath and seeded in a 75T flask so that the cell count would be 5 × 10⁶ cells. After culturing for 1 to 3 days, the cells were retrieved and seeded in each well of a 24-well plate so that the cell count would be 0.5 × 10⁵ cells/well. Cells in each well in a 60 to 80% confluent state after culturing for 1 to 3 days were transfected with about 1 µg of each plasmid DNA described above using 3 µl of Lipofectamine 2000 (Life Technologies, Carlsbad, USA).

### <Sequencing>

The frequency and location of mutations were analyzed by retrieving cultured cells 24 to 72 hours after transfection, extracting a DNA, amplifying a target region by PCR, then performing amplicon analysis using a next generation sequencer Miniseq. Data was processed with CLC Workbench. In some cases, construct expressing cells were fractionated with a cell sorter by using fluorescence of GFP or RFP as an indicator for concentration of cells.

### Example 1: Investigation of making a cytidine deaminase compact by deletion of a terminal region

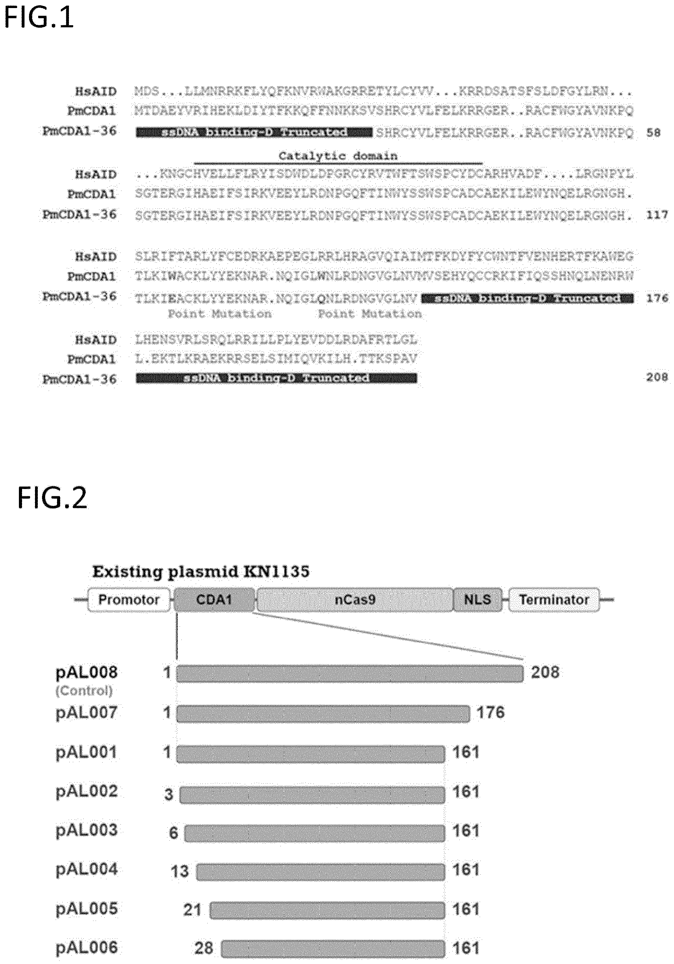

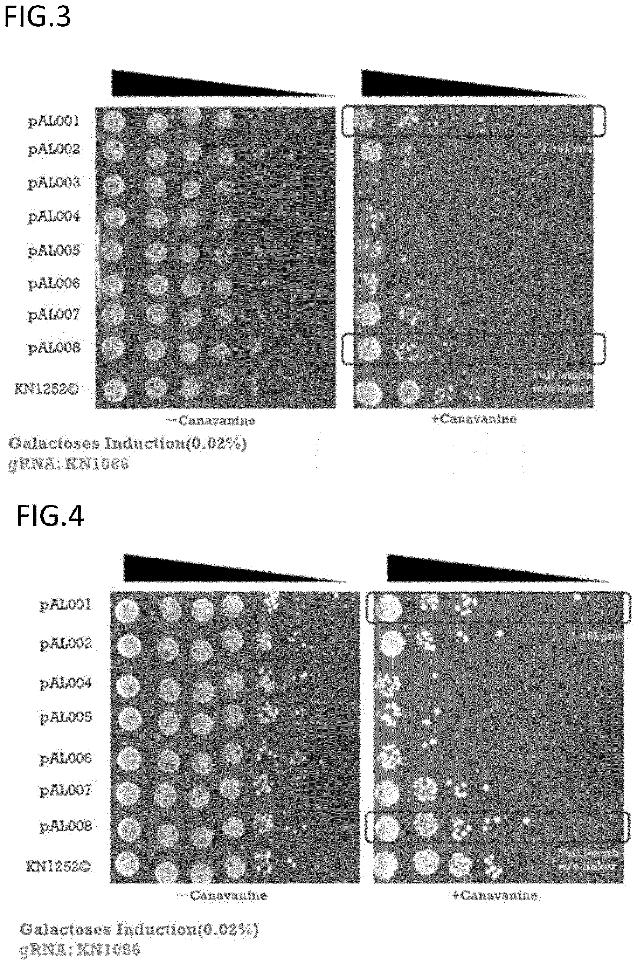

The nucleic acid altering enzyme complex comprising a compact cytidine deaminase, disclosed in Non Patent Literature 4, comprises a uracil-DNA glycosylase inhibitor (UGI) . Meanwhile, it is expected that an undesired off-target effect is enhanced if a UGI is used. When comparatively evaluating deaminase activity, the mutation introduction efficiency is saturated and the difference is difficult to discern in the presence of a UGI within yeast cells. For this reason, a complex with a UGI removed was first prepared from the complex disclosed in Non Patent Literature 4 described above. A complex with further deletion of an N-terminus side region thereof was also prepared. The efficiency of altering a target site in these complexes was investigated. Figure 2 shows the summary of the constructs used in this experiment, and Figures **3** to **5** show the results. Unexpectedly, even for a complex using CDA1Δ161 which was found to have high efficiency of alteration in Non Patent Literature 4, the efficiency of alteration was 2/3 relative to a complex using wild-type CDA1 when a UGI was not used in view of Figures **3** to **5****.** Furthermore, alteration decreased significantly in CDA1 with 2 or more amino acid residue deletions on the N-terminus side of CDA1Δ161. In view of the results described above, the inventors reached a conclusion that the complex disclosed in Non Patent Literature 4 was able to achieve high efficiency of altering a targeted site largely due to an effect of improving efficiency of DNA alteration by a UGI within yeast cells, and a complex that can achieve a desired efficiency of alteration cannot be obtained simply by deleting a terminal region of CDA1 when a UGI is not used or when applied to a different organism species.

### Example 2: Investigation of making cytidine deaminase compact based on a three-dimensional structure

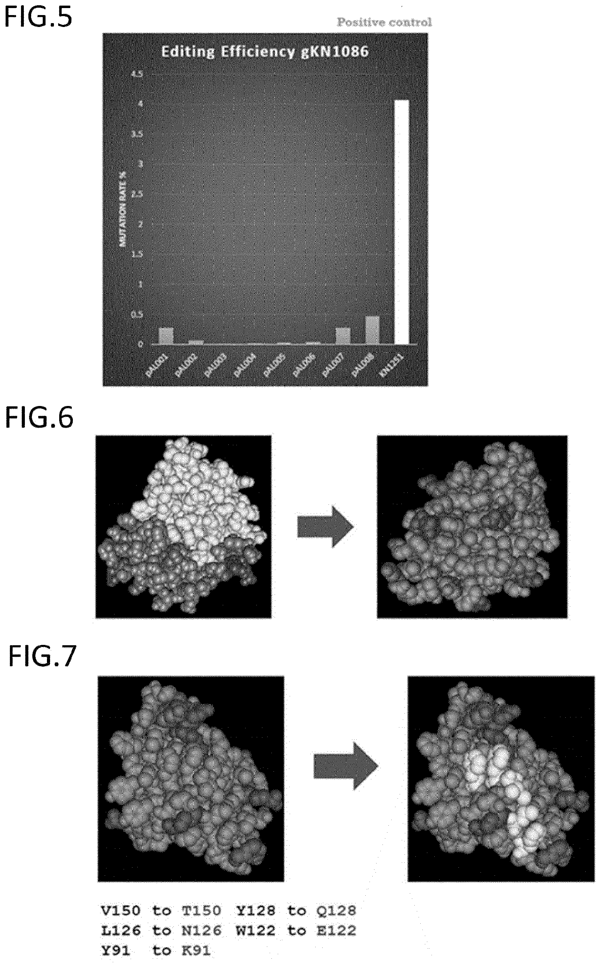

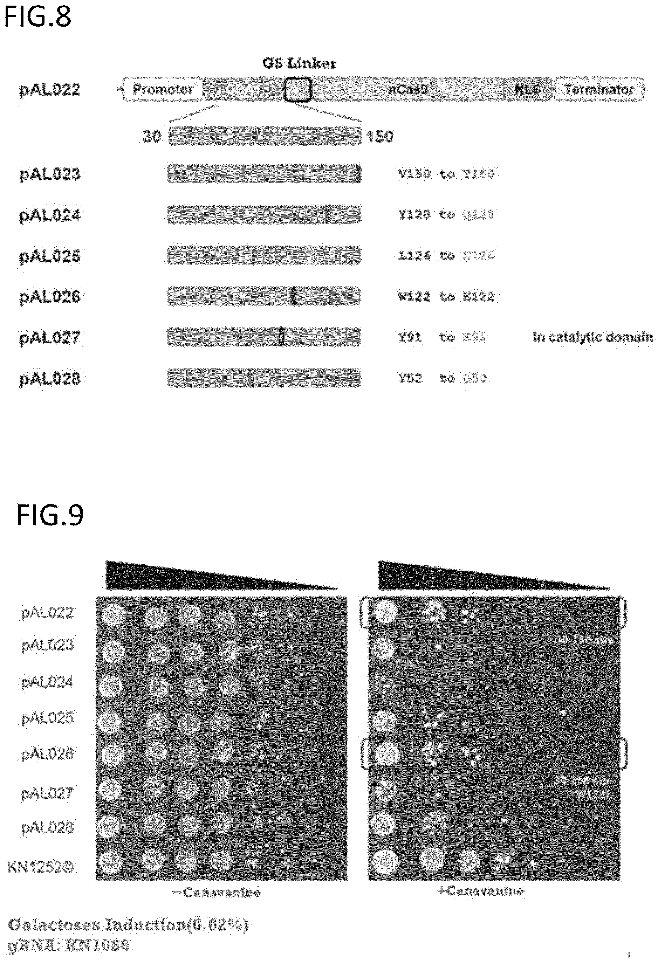

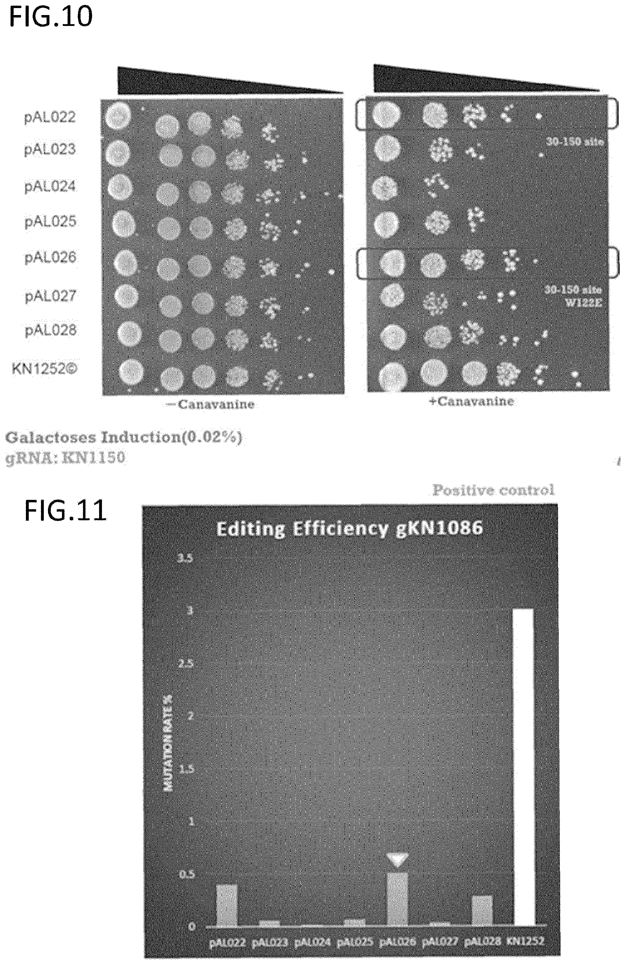

In this regard, a site of deletion in a cytidine deaminase was determined based on the structure of the cytidine deaminase instead of simply deleting the terminal region of the cytidine deaminase. First, the structure of CDA1Δ161 was analyzed using a three-dimensional structure analysis software, which revealed that the structure had a distorted spherical shape (left diagram of Figure **6**). In this regard, only the white portion in the left diagram of Figure **6** (e.g., regions of positions 30 to 150 of PmCDA1) was extracted to approximate the shape to a more spherical shape. Hereinafter, the positions of amino acid residues are indicated based on the amino acid sequence of a wild-type cytidine deaminase (i.e., sequence set forth in SEQ ID NO: 1). A compact cytidine deaminase prepared in this manner (also referred to as PmCDA1 (30-150)) was subjected to further structural analysis to prepare a complex with exposed internal amino acid residues (amino acid residues at positions 91, 122, 126, 128, and 150 in this case) (white portions in the right diagram of Figure **7****)** substituted from hydrophobic to hydrophilic amino acid residues. Figure **8** shows the summary of the constructs used in this experiment, and Figures **9** to **11** show the results. Figures **9** to **11** show that the efficiency of altering a target site is improved with PmCDA1 (30-150) relative to CDA1Δ161 having a high molecular weight of cytidine deaminase. A particularly high alteration activity was observed in a cytidine deaminase which is PmCDA1 (30-150) having tryptophan at position 122 substituted with glutamic acid (also referred to as (PmCDA1 (30-150; W122E)).

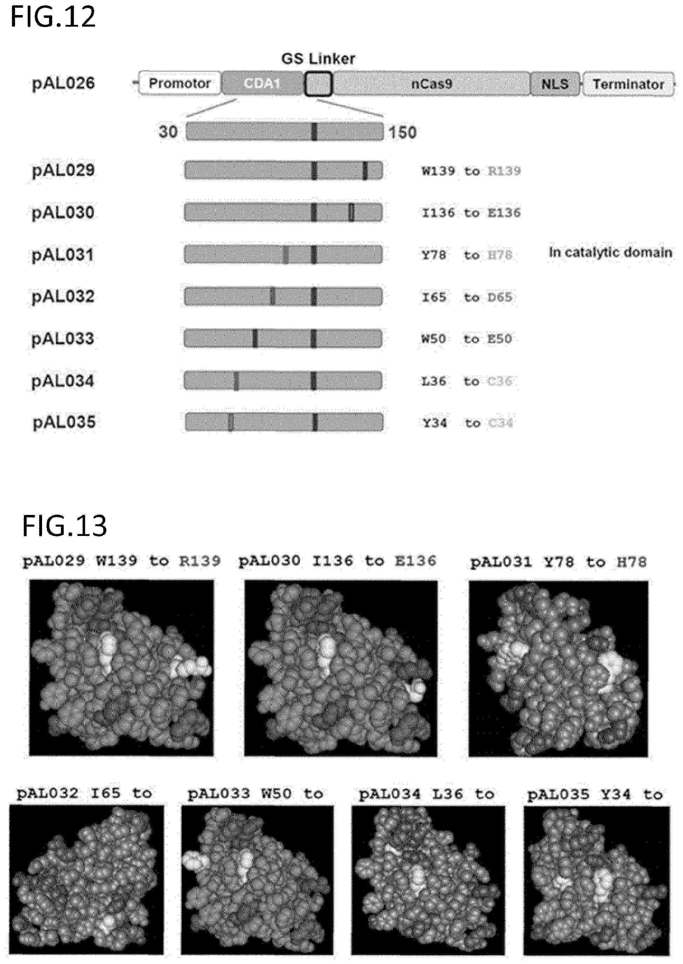

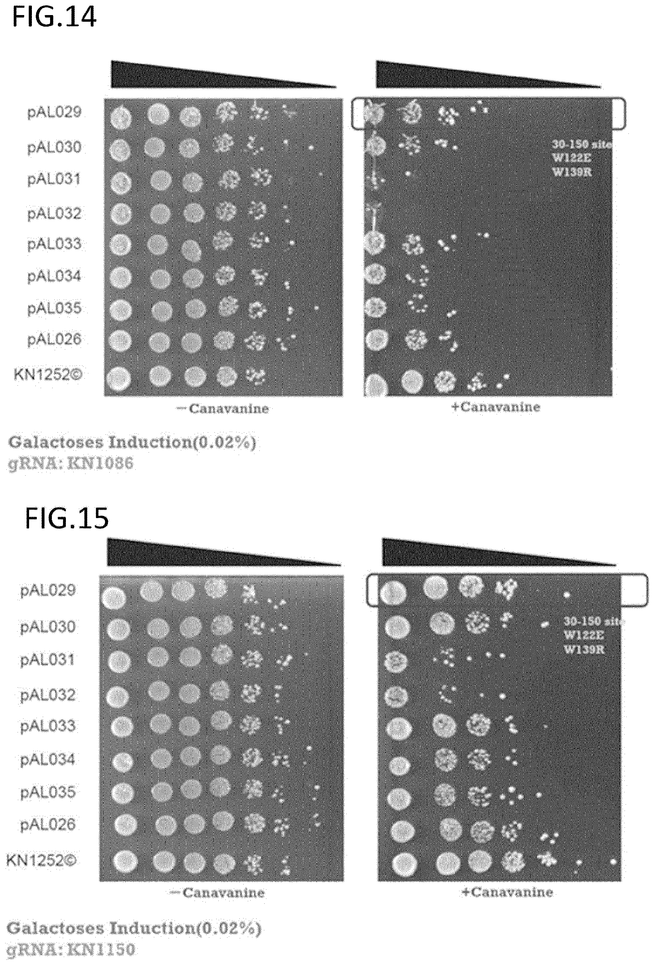

Furthermore, efficiency of altering a target site was investigated by using a complex in which a hydrophobic amino acid is substituted with a hydrophilic amino acid in PmCDA1 (30-150; W122E). Figure **12** shows the summary of the constructs used in this experiment, Figure **13** shows a portion introduced with a mutation in a three-dimensional structure (white portion), and Figures **14** to **16** show the results. A particularly high alteration activity was observed in a cytidine deaminase which is PmCDA1 (30-150; W122E) with tryptophan at position 139 substituted with arginine (also referred to as PmCDA1 (30-150; W122E; W139R)) from Figures **14** to **16****.**

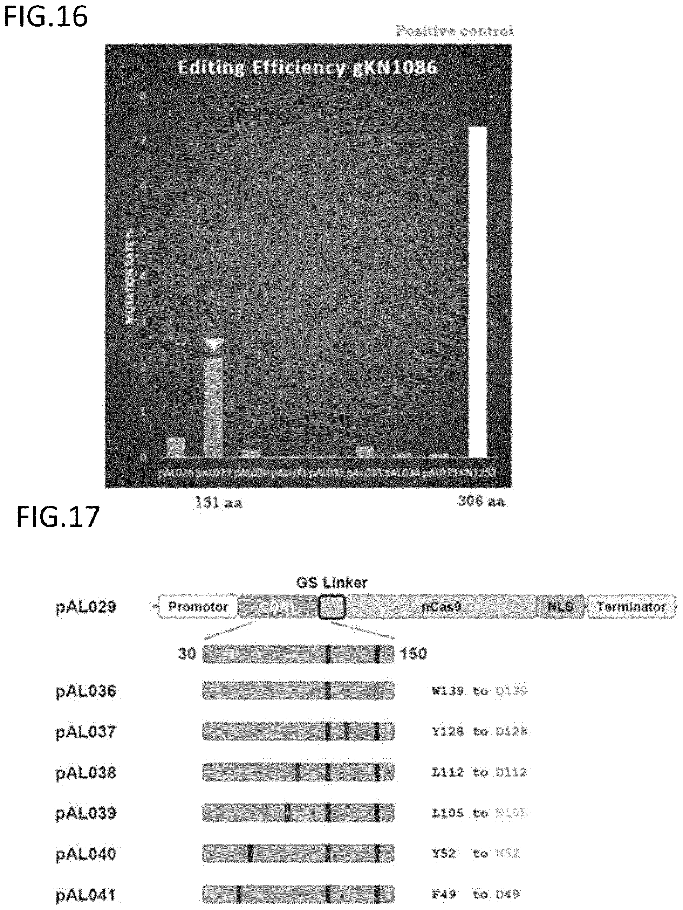

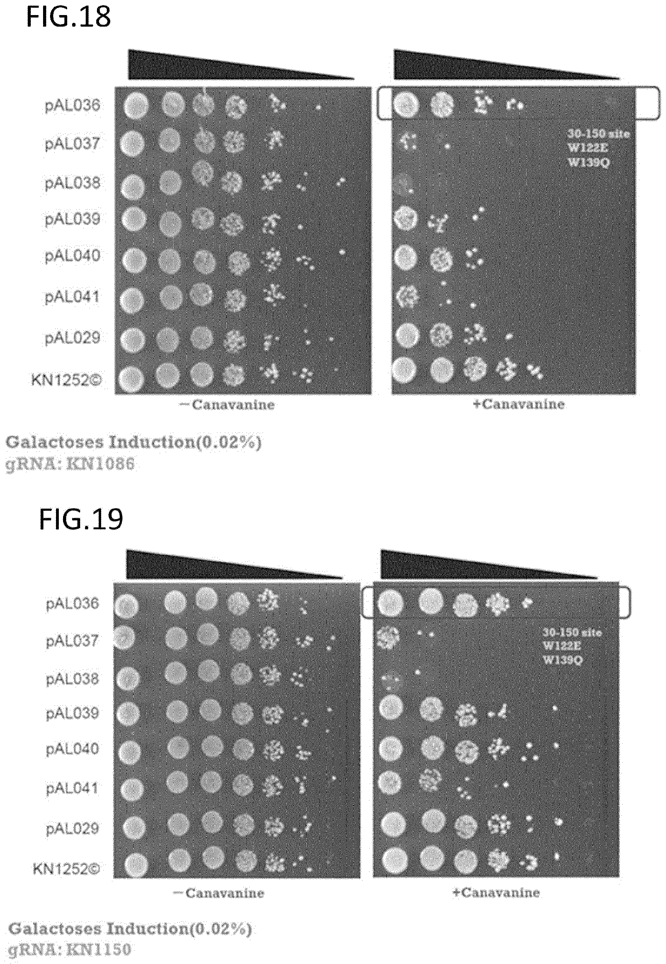

Similarly, a complex with the location of mutation changed was prepared to investigate the efficiency of altering a target site. Figure **17** shows the summary of the constructs used in this experiment, and Figures **18** to **20** show the results. A particularly high alteration activity was observed in a cytidine deaminase which is PmCDA1 (30-150; W122E) with tryptophan at position 139 substituted with glutamine (also referred to as PmCDA1 (30-150; W122E; W139Q)) from Figures **18** to **20****.**

### Example 3: Investigation of efficiency of altering a nucleic acid altering enzyme complex using split SpCas9

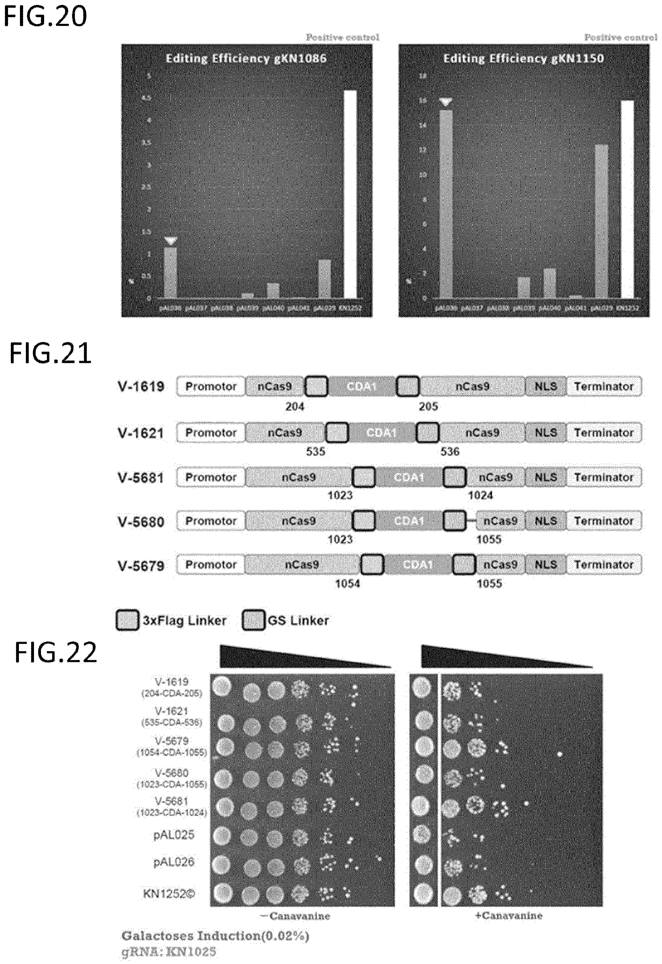

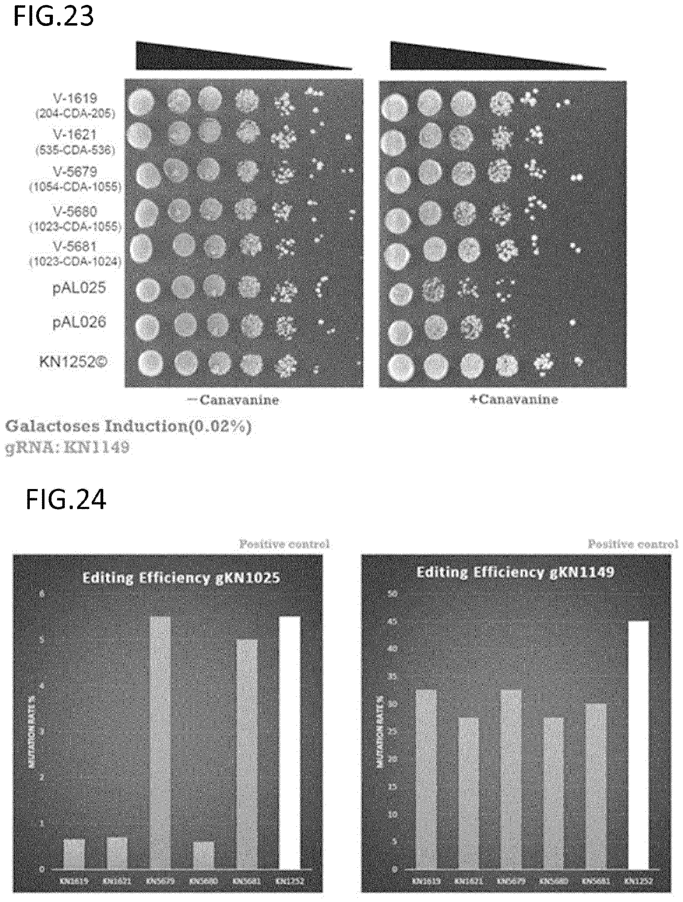

Whether efficiency of altering a nucleic acid altering enzyme complex can be improved was investigated by a completely different approach. Conventional nucleic acid altering enzyme complexes with a cytidine deaminase fused to a terminus of a Cas protein are used. Meanwhile, a demonstration experiment was conducted under the hypothesis that stability as a complex would improve and access to a substrate DNA would improve by embedding a cytidine deaminase inside a Cas protein. Figure **21** shows the summary of the constructs used in this experiment, and Figures **22** to **24** show the results. High efficiency of alteration was observed in all prepared complexes in view of Figures **22** to **24****.**

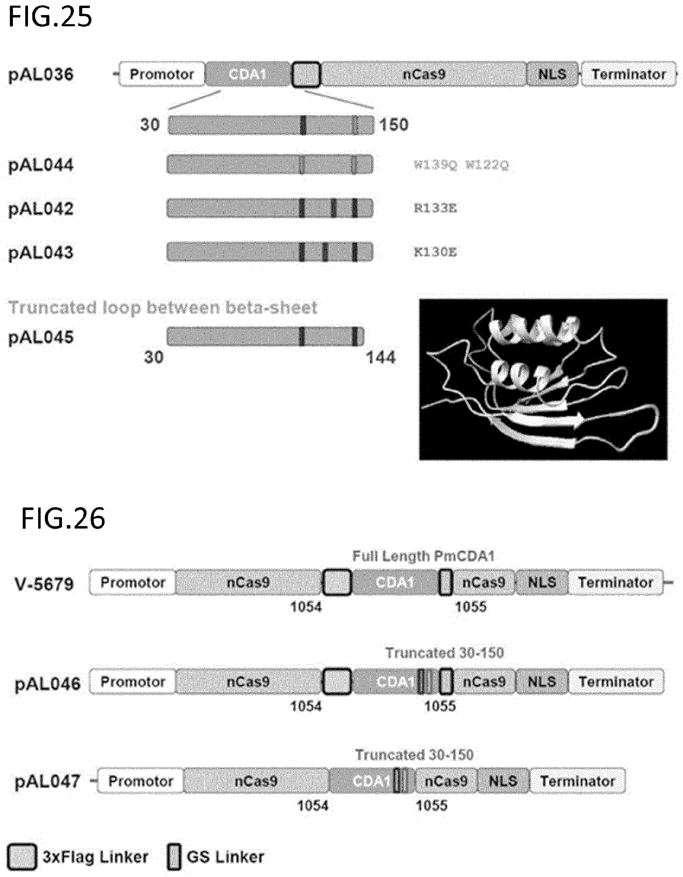

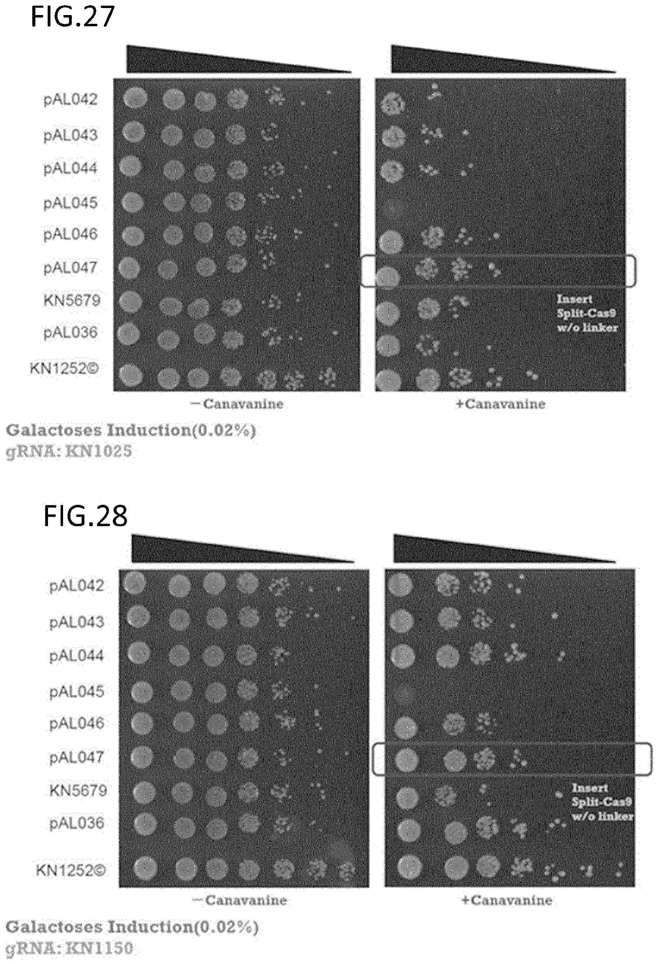

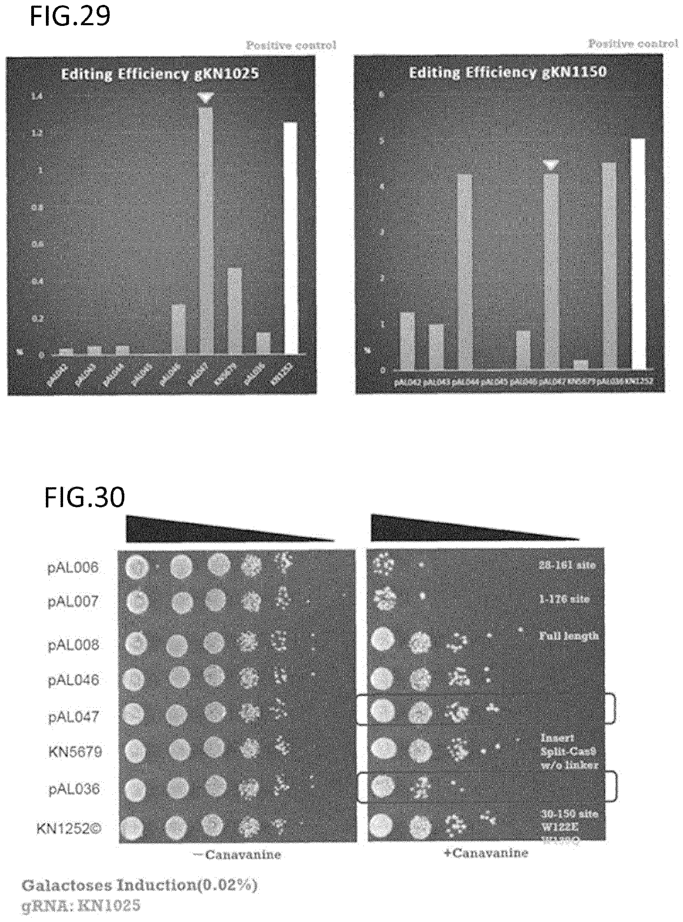

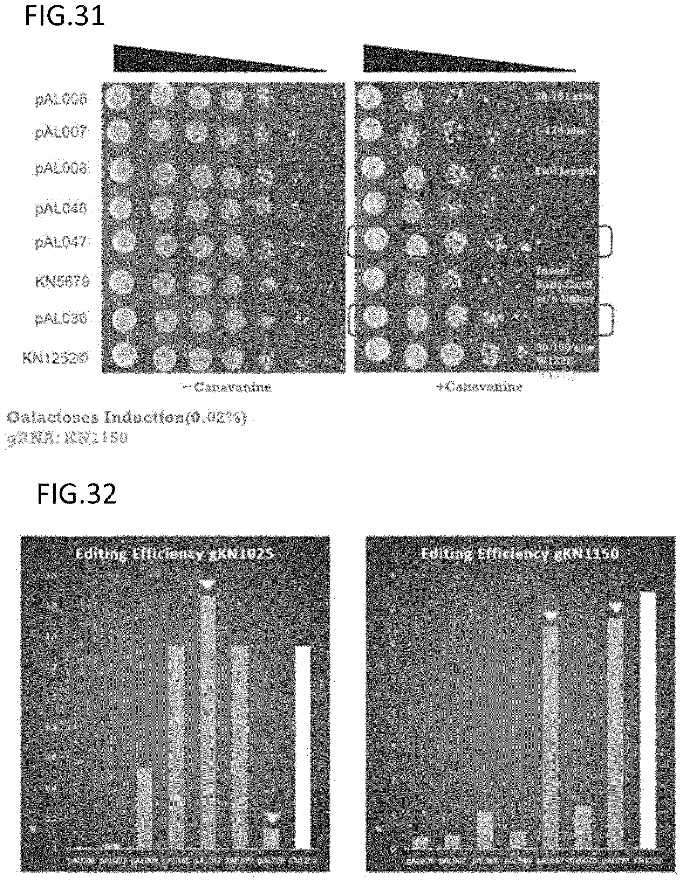

Next, efficiency of altering a complex was investigated by using a compact cytidine deaminase similar to those investigated in Examples 2 in place of a wild-type cytidine deaminase. This experiment used PmCDA1 (30-150; W122Q; W139Q), PmCDA1 (30-150; W122E; K133E; W139R), PmCDA1 (30-150; W122E; K130E; W139R), and PmCDA1 (30-144; W122E; W139R) with deletion of a loop region (region of 145 to 150) between β sheets as the compact cytidine deaminase. Furthermore, efficiency of altering a complex was investigated by using a complex having a linker between an N-terminal fragment and C-terminal fragment of a Cas9 protein and a compact cytidine deaminase (PmCDA1 (30-150; W122E; W139Q)) and a complex without a linker. Figures **25** and **26** show the summary of the constructs used in this experiment, and Figures **27** to **32** show the results. A particularly high efficiency of alteration was observed in a complex having PmCDA1 (30-150; W122E; W139Q) as a cytidine deaminase and not having a linker between an N-terminal fragment and C-terminal fragment of a Cas9 protein and said cytidine deaminase in view of Figures **27** to **32****.**

### Example 4: Investigation of efficiency of altering a nucleic acid altering enzyme complex having a compact cytidine deaminase and a UGI

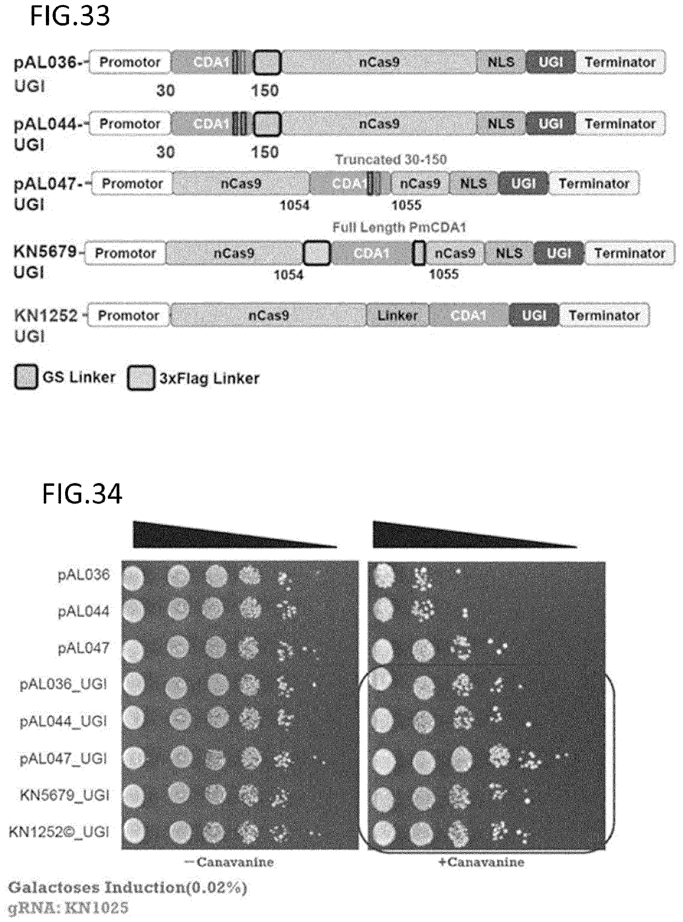

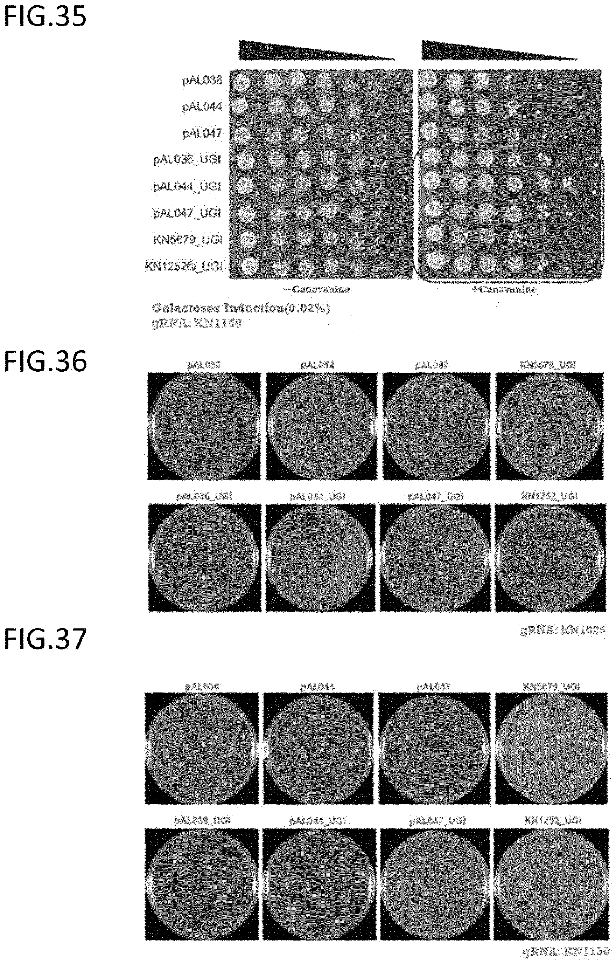

A UGI was further used concurrently with the complex found to have high efficiency of alteration in Examples 2 and 3 described above to investigate whether efficiency of alteration improves. Figure **33** shows the summary of the constructs used in this experiment, and Figures **34** and **35** show the results. High efficiency of alteration was observed in all prepared complexes in view of Figures **34** and **35****.**

### Example 5: Investigation of off-target effect of a nucleic acid altering enzyme complex

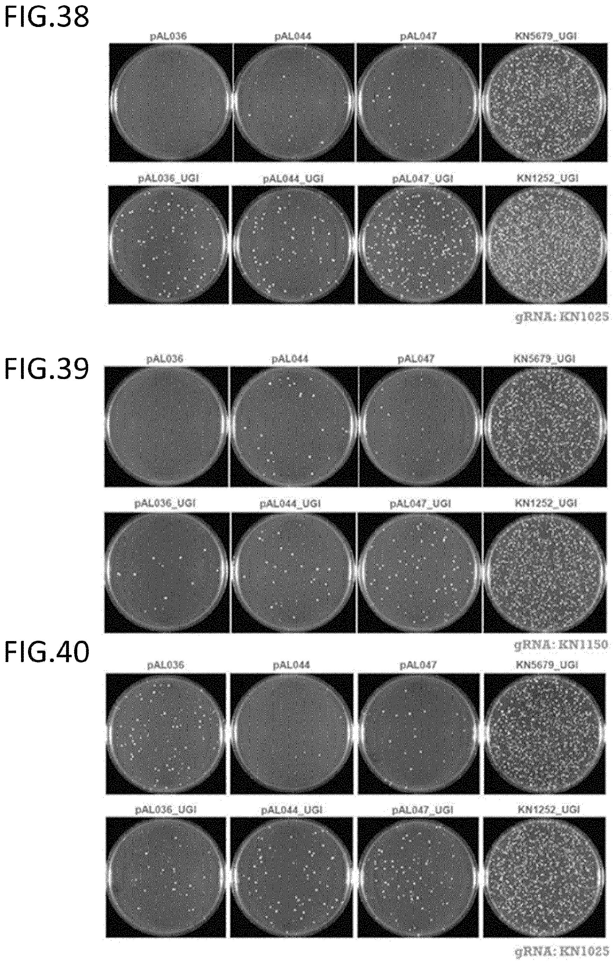

An off-target effect was investigated for the complexes found to have high efficiency of alteration in the Examples described above. Figures **36** to **41** show the results. An off-target effect was suppressed more significantly in all prepared complexes compared to a control (using KN1252_UG1) in view of the results.

### Example 6: Investigation of alteration using compact Cas9 (SaCas9)

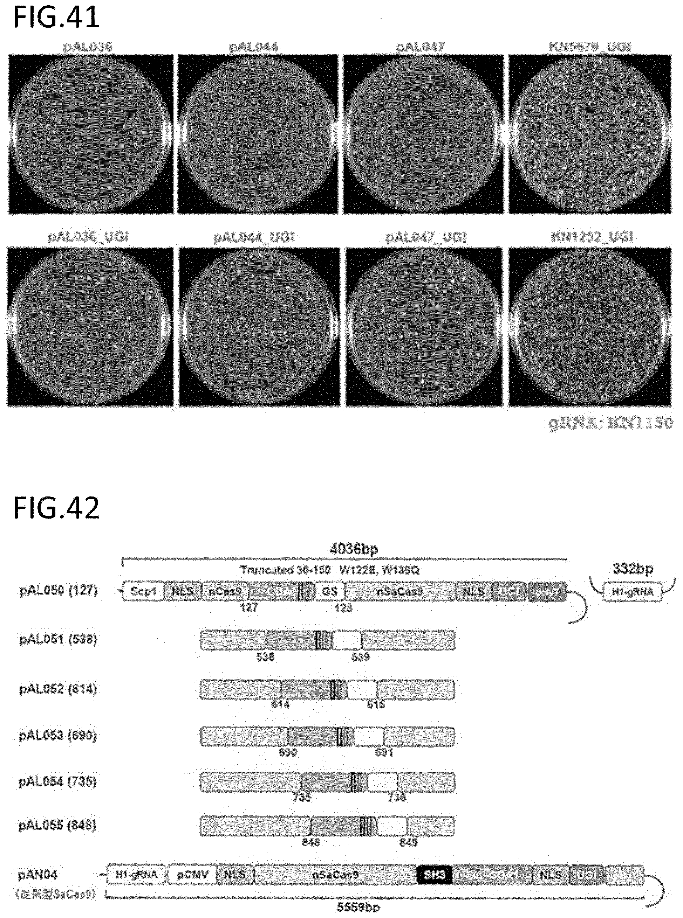

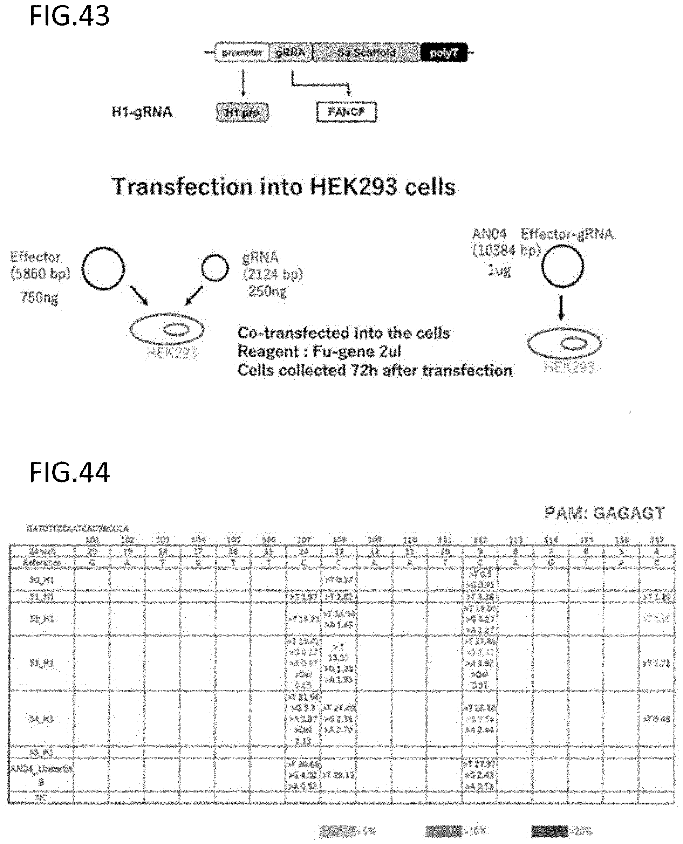

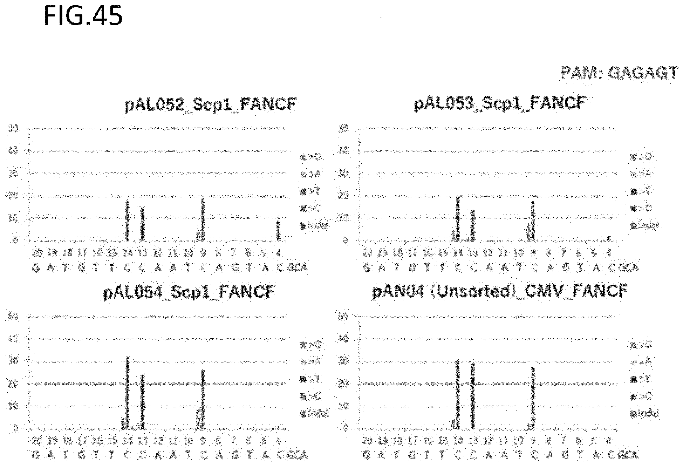

Next, whether the same alteration is observed by using SaCas9 in place of SpCas9 in Example 3 was investigated. Use of SaCas9 and a compact deaminase enabled an expression cassette of a nucleic acid altering enzyme complex including a guide RNA expression cassette (consisting of a promoter, gRNA encoding sequence, and polyT sequence) to have a size that can be inserted into an AAV vector (about 4.4 kb) or less. Figure **42** shows the summary of the constructs used in this experiment (nucleic acid altering enzyme complex), top diagram of Figure **43** (guide RNA) and bottom diagram of Figure **43** show the summary of the experimental procedure, and Figures **44** to **45** show the results. High efficiency of alteration was observed in all prepared complexes in view of Figures **44** and **45****.** Suppression of an off-target effect is also expected. Interestingly, it was demonstrated that a site with a high probability of being introduced with a mutation varies depending on the portion of inserting a deaminase into Cas9. Thus, a site where a mutation in introduced can be adjusted by adjusting the site of insertion described above.

### Example 7: Recovery of deaminase activity and removal of a DNA binding region of PmCDA1

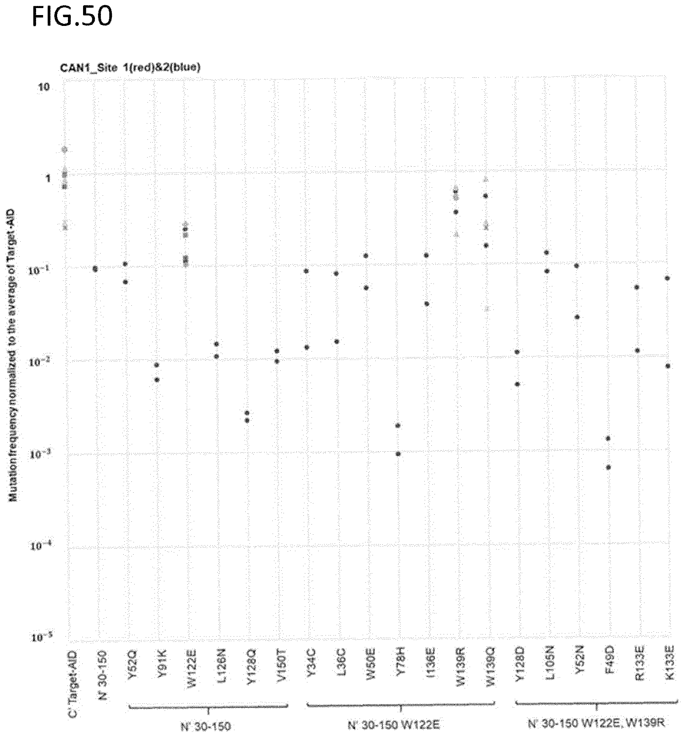

A DNA deaminase has affinity unique to a DNA and induces non-specific deamination. It has been elucidated to form a complex with a double stranded DNA at a different region from a catalytic core in view of the structure of hAID, which is a human homolog of PmCDA1 (Figure **46a****).** A latent DNA biding site of PmCDA1 was located at residues 21 to 27 and 172 to 192 among the full length of 208 amino acids of a protein in view of the amino acid sequences of hAID and PmCDA1 (Figure **46a**). To delete an expected DNA binding region, a series of cleaved fragments (1-201, 1-197, 1-190, 1-183, 1-179, 1-176, and 1-161) was first prepared from the C-terminus, and base editing activity was tested with yeast *Saccharomyces cerevisiae* (BY4741) cells (Figure **48****).** While it has been reported that PmCDA1 (1-161) from cleaving 47 amino acids exhibits the same editing efficiency as the full length PmCDA1 (1-208) in yeasts, activity gradually decreased with more cleavage for those fused to the C-terminus of nCas9 without an addition of a uracil-DNA glycosylase inhibitor (UGI). Next, a series of cleavages was performed from the N-terminus of 1 to 161 residues with fusion to the N-terminus of nCAs9. In an N-terminus cleaved fragment of CDA1 (1-161), activity initially decreased further, but recovered thereafter once cleavage proceeded to 21 and 28 amino acids (Figure **49****).** It was found from the predicted structure of CDA1 that simultaneous cleavage of the N-terminus and the C-terminus minimizes the cross-sectional area, resulting in a smooth protein surface with exposure of few hydrophobic residues (Figure **49****).** Furthermore, activity recovered when cleaved down to CDA1 (30-150) (Figures **46b** and **49****),** which is predicted to be the smallest, with exposure of the hydrophobic surface suppressed to the minimum while maintaining the enzyme core domain (Figure **49**). These results suggest that the change in editing activity is due to the stability of the conformation of the protein. Furthermore, a series of mutations was introduced to hydrophobic residues exposed after cleavage in order to further improve the activity thereof. When six mutations were initially tested, W122E was found to impart significant activity to CDA1 (30-150) (Figure **50****).** When seven mutations were combined with W122E and tested, W133R/Q, which further improved the activity, was found (Figure **50****).** Hereinafter, CDA1 (30-150) comprising W122E and W133Q is referred to as tCDA1EQ.

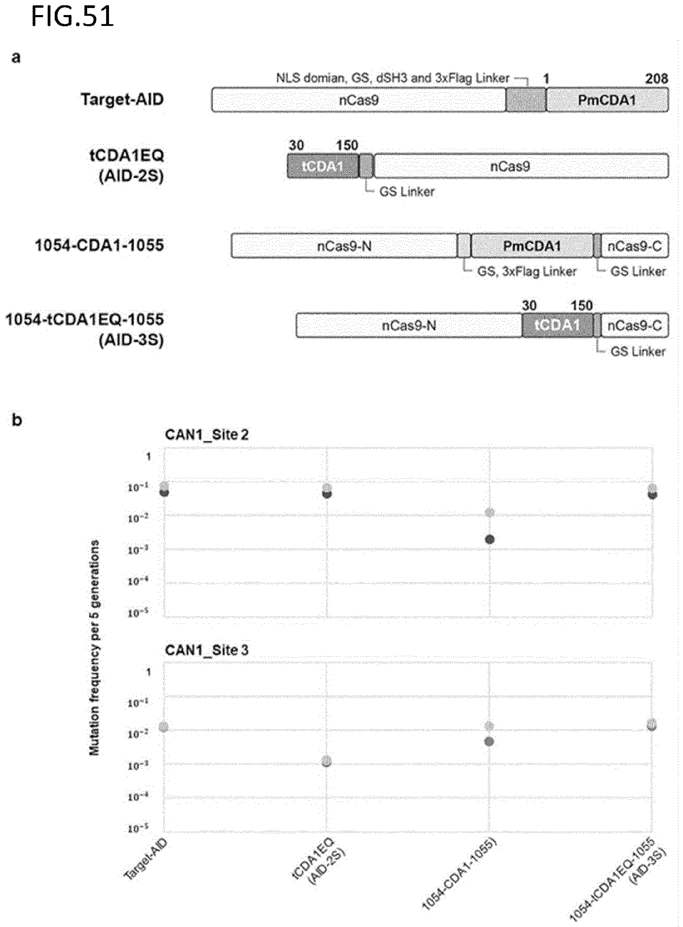

Since it is understood that the altered deaminase has lower affinity to a DNA and lower stability than the original PmCDA1, the nCas9 fusion structure may have a large impact on the base editing property. Besides fusion to a terminus of nCas9, an nCas9 polypeptide can be split and both termini of a protein can be fused to the split portions to embed a deaminase in the middle. Structurally, the position of 1054 amino acid in the RuvC domain of Cas9 is on a flexible protein surface, and is close to a non-target DNA strand subjected to deamination. While variation in editing efficiency was observed between target sites evaluated in a CAN1 assay for tCDA1EQ fused to the N-terminus, consistent editing efficiency equivalent to that of the original Target-AID was exhibited in an embedded form (Figures **46d** and **51****).**

In order to evaluate an off-target effect which is non-specific and independent of gRNA, instances of thialysine resistant mutants were measured for artificial complexes fused to a UGI (LYP1 assay). It was found that both N-terminus fused form and embedded form of tCDA1EQ complexes have significantly reduced rate of manifestation of mutations relative to the original Target-AID (5 to 79-fold) (Figure **47a****)** and significantly reduced gRNA independent off-target effects. These N-terminus fused form and embedded form of tCDA1EQ complexes were named AID-2S (Small and Specific) and AID-3S (Small, Specific, and Superior), respectively.

### Example 8: Evaluation of AID-2S and AID-3S in mammalian cells

Next, the editing efficiency and window of AID-2S and AID-3S in human HEK293T cells were evaluated and compared with existing improved cytosine base editors YE1, YE2, and R33A+K34A reported to have reduced off-target effects. Four well studied on-target sites (HEK2, HEK3, RNF2, and VEGFA) were edited by transfection with a plasmid DNA vector and analyzed by amplicon deep sequencing. Target-AID, AID-2S, and YE1 exhibited consistently high efficiency on all four tested target sites. AID-3S and YE2 exhibited moderate to high efficiency depending on the target site. R33A+K34A had poor efficiency at a target site of HEK3 (Figure **46e****).** The average editing window width of AID-2S was narrower than Target-AID and similar to YE1 and YE2 (Figure **46f****).**

The gRNA independent off-target effect was evaluated by an orthogonal SaCas9 R-loop assay by using HEK293T cells (Figure **47b****).** SaCas9 off-target sites 1 to 6 were selected based on previous studies, and site 7 (VEGFA locus) was selected because of the possibility of C-rich context exhibiting high sensitivity to deamination by a CBE. Target-AID exhibited detectable off-target editing at all seven sites (Figure **47c****).** Meanwhile, AID2S did not have detectable off-target editing at sites 1 and 3, and off-target editing decreased significantly at sites 2, 5, 6, and 7, resulting in the same results as YE2 and R33A+K34A. YE1 exhibited a slightly high off-target editing at sites 6 and 7. AID-3S was the lowest at seven sites and hardly detected. It is understood that this is due to the loss of affinity to a DNA and three-dimensional restriction of access of an enzyme to sites other than a DNA strand to which Cas9 is bound. AID-2S and -3S reduced off-target editing of an R-loop relative to the original Target-AID by an average of about 4.5-fold and 13.7-fold, but the efficiency of on-target editing was mostly maintained (Figures **47c** and **47d****).** These results, together with a yeast LYP1 assay, consistently support that off-target effects independent of gRNA of the entire genome are significantly reduced in AID-2S and -3S. Furthermore, a gRNA dependent off-target effect was studied by deep sequencing of the six reported sites (HEK2_OF1, 2; VEGFA_OF1, 2, 3, 4) (Figure **47e****).** Off-target editing was significantly reduced at all analyzed sites for AID-2S and AID-3S, and YE2 and R33A+K34A.

### Example 9: Minimization of a cytosine base editing system

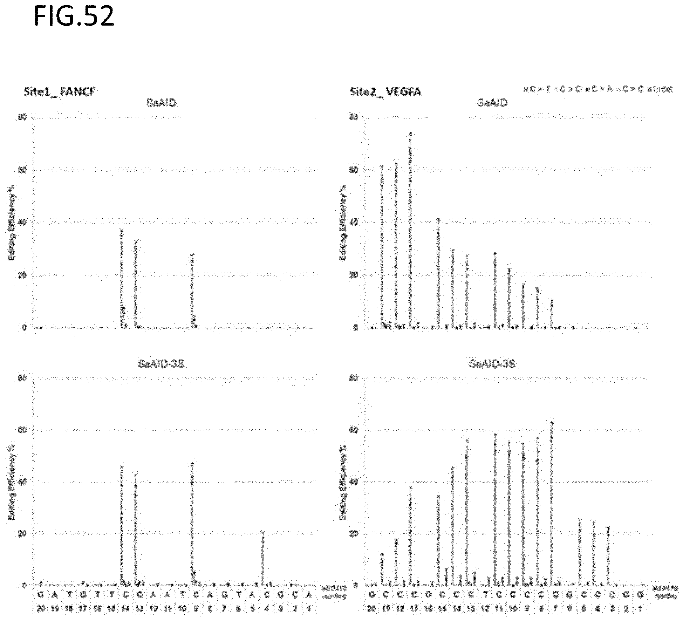

Altered PmCDA1 (tCDA1EQ) is significantly smaller in size (121 amino acids) compared to wild-type (208 amino acids). A small molecule size as a genome editing component is particularly advantageous for in vivo delivery tools such as an AAV vector where the length of DNA is restricted to 4 to 5 kb. Even if a compact SaCas9 system is used, the size would be clearly beyond the limit if a base editing component is added (Figure **46g****).** In this regard, tCDA1EQ was incorporated into the positions of residues 615 to 616 of nSaCas9 within an HNH domain facing a polynucleotide binding cleft to develop SaAID-3S comprising all required base editing components at a size that can be inserted into an AAV vector. gRNA expression cassettes with a full length of 4036 bp and 332 bp were constructed using a compact Scp1 promoter and an SpA terminator. For comparison, a conventional SaCas9 version of Target-AID (SaAID) was also developed. For this SaAID, a linker, UGI, CMV promoter, and SV40 terminator were added to the full length PmCDA1 for a full length of 5220 bp, while a gRNA cassette was not added. To normalize the transfection efficiency that varies by the size of a vector, transfected cells were sorted out by a fluorescent signal of iRFP670 expressed from a vector backbone. Both constructs exhibited the same editing efficiency (Figure **46h****)** albeit with a difference in the mutation window (Figure **52****)** at two tested target sites.

Wide-ranging application from breeding of plants or microorganisms to clinical applications is expected by minimizing the off-target effect and enhancing on-target editing. AID-3S is also demonstrated in SaCas9 orthologs, provides a minimum base editing system of the same size which can be inserted into a single AAV vector, and facilitates application to safer gene therapy.

### (Note)

As described above, the present disclosure is exemplified by the use of its preferred embodiments. It is understood that the scope of the present disclosure should be interpreted solely based on the claims. It is understood that any patent, any patent application, and any references cited herein should be incorporated herein by reference in the same manner as the contents are specifically described herein. The present application claims priority to Japanese Patent Application No. 2020-149419 filed with the JPO on September 4, 2020. The entire content thereof is incorporated herein by reference in the same manner as the contents constitutes the present application in its entirety.

### [Industrial Applicability]

The present disclosure provides a complex for altering a double stranded DNA, which is more compact, has higher efficiency of alteration, and has an off-target effect that is more suppressed compared to conventional technologies. A nucleic acid encoding such a complex can also be inserted into an adeno-associated viral vector and facilitate delivery of a complex to a target site, so that such a nucleic acid can be useful especially in application to gene therapy, etc.

## Claims

1. A complex of a nucleic acid sequence recognition module bound with a deaminase, wherein
the nucleic acid sequence recognition module specifically binds to a target nucleotide sequence in a double stranded DNA,
the deaminase is altered so that the deaminase has a smaller size than a wild-type deaminase corresponding to the deaminase, and an area of a cross-section exposed as a result of alteration or an index indicating the area is less than or equal to a predetermined value, and
the complex has an ability to alter a targeted site of the double stranded DNA.

2. The complex of claim 1, wherein the deaminase is altered so that the number of hydrophobic amino acid residues manifested on a cross-section exposed as a result of altering the deaminase is less than or equal to a predetermined value, and the alteration comprises a deletion.

3. The complex of claim 1 or 2, wherein the deaminase is altered so that a ratio of the number of hydrophobic residues manifested on a cross-section exposed as a result of alteration to the number of altered amino acid residues is less than or equal to a predetermined value, and the alteration comprises a deletion.

4. The complex of any one of claims 1 to 3, wherein the deaminase is altered so that the number of hydrophobic amino acid residues manifested on a cross-section exposed as a result of altering the deaminase is minimized.

5. The complex of any one of claims 1 to 4, wherein the deaminase is altered so that a ratio of the number of hydrophobic residues manifested on a cross-section exposed as a result of alteration to the number of altered amino acid residues is minimized.

6. The complex of any one of claims 1 to 5, wherein the deaminase is from altering an N-terminus side and a C-terminus side of the wild-type deaminase.

7. The complex of any one of claims 1 to 6, wherein at least one hydrophobic internal amino acid residue exposed in the deaminase is substituted with a hydrophilic amino acid residue.

8. The complex of any one of claims 1 to 7, wherein the deaminase comprises a cytidine deaminase.

9. The complex of any one of claims 1 to 8, wherein the deaminase consists of:
(1) an amino acid sequence consisting of a region of amino acid residues at positions 30 to 150 in the amino acid sequence set forth in SEQ ID NO: 1;
(2) an amino acid sequence consisting of a region corresponding to the region of (1) which is an ortholog of a protein consisting of the amino acid sequence set forth in SEQ ID NO: 1;
(3) the amino acid sequence of (1) or (2) with one or several amino acid deletions, substitutions, insertions, and/or additions; or
(4) an amino acid sequence with 90% or greater similarity or identity with the amino acid sequence of (1) or (2).

10. The complex of claim 9, wherein the amino acid sequence of (3) comprises one or more substitutions of an amino acid residue at a position selected from the group consisting of positions 122, 126, and 139 in the amino acid sequence set forth in SEQ ID NO: 1 or an amino acid residue corresponding to said position to a hydrophilic amino acid residue.

11. The complex of claim 9 or 10, wherein the amino acid sequence of (3) comprises two or more substitutions of an amino acid residue at position 122 and an amino acid residue at position 139 in the amino acid sequence set forth in SEQ ID NO: 1 or amino acid residues corresponding to said positions to hydrophilic amino acid residues.

12. The complex of any one of claims 1 to 11, wherein the nucleic acid sequence recognition module is selected from the group consisting of a CRISPR-Cas system wherein at least one DNA cleaving capability of a Cas protein is inactivated, a zinc finger motif, a TAL effector, and a PPR motif.

13. The complex of any one of claims 1 to 12, wherein the nucleic acid sequence recognition module is a CRISPR-Cas system wherein at least one DNA cleaving capability of a Cas protein is inactivated.

14. The complex of claim 12 or 13, wherein the Cas protein is a Cas9 protein.

15. A complex of an N-terminal fragment of a nucleic acid sequence recognition module, a deaminase, and a C-terminal fragment of a nucleic acid sequence recognition module bound therewith, wherein
when the N-terminal fragment and the C-terminal fragment of the nucleic acid sequence recognition module are refolded, the nucleic acid sequence recognition module has an ability to specifically bind to a target nucleotide sequence in a double stranded DNA and alter a targeted site of the double stranded DNA.

16. The complex of claim 15, wherein the deaminase is altered so that the deaminase has a smaller size than a wild-type deaminase corresponding to the deaminase, and an area of a cross-section exposed as a result of alteration or an index indicating the area is less than or equal to a predetermined value.

17. The complex of claim 15 or 16, wherein the deaminase is altered so that the number of hydrophobic amino acid residues manifested on a cross-section exposed as a result of altering the deaminase is less than or equal to a predetermined value, and the alteration comprises a deletion.

18. The complex of any one of claims 15 to 17, wherein the deaminase is altered so that a ratio of the number of hydrophobic residues manifested on a cross-section exposed as a result of alteration to the number of altered amino acid residues is less than or equal to a predetermined value, and the alteration comprises a deletion.

19. The complex of any one of claims 15 to 18, wherein the deaminase is altered so that the number of hydrophobic amino acid residues manifested on a cross-section exposed as a result of altering the deaminase is minimized.

20. The complex of any one of claims 15 to 19, wherein the deaminase is altered so that a ratio of the number of hydrophobic residues manifested on a cross-section exposed as a result of alteration to the number of altered amino acid residues is minimized.

21. The complex of any one of claims 15 to 20, wherein the deaminase is from altering an N-terminus side and a C-terminus side of the wild-type deaminase.

22. The complex of any one of claims 15 to 21, wherein at least one hydrophobic internal amino acid residue exposed in the deaminase is substituted with a hydrophilic amino acid residue.

23. The complex of any one of claims 15 to 22, wherein the deaminase comprises a cytidine deaminase.

24. The complex of any one of claims 15 to 23, wherein the deaminase consists of:
(1) an amino acid sequence consisting of a region of amino acid residues at positions 30 to 150 in the amino acid sequence set forth in SEQ ID NO: 1;
(2) an amino acid sequence consisting of a region corresponding to the region of (1) which is an ortholog of a protein consisting of the amino acid sequence set forth in SEQ ID NO: 1;
(3) the amino acid sequence of (1) or (2) with one or several amino acid deletions, substitutions, insertions, and/or additions; or
(4) an amino acid sequence with 90% or greater similarity or identity with the amino acid sequence of (1) or (2).

25. The complex of any one of claims 15 to 24, wherein the nucleic acid sequence recognition module is selected from the group consisting of a CRISPR-Cas system wherein at least one DNA cleaving capability of a Cas protein is inactivated, a zinc finger motif, a TAL effector, and a PPR motif.

26. A nucleic acid encoding the complex of any one of claims 1 to 25.

27. A vector comprising the nucleic acid of claim 26.

28. The vector of claim 27, which is an adeno-associated viral vector.

29. A method of altering a targeted site of a double stranded DNA of a cell, comprising contacting the complex of any one of claims 1 to 25 with the double stranded DNA.

30. The method of claim 29, wherein contacting a double stranded DNA with a complex is performed through introduction of the nucleic acid or vector of any one of claims 26 to 28 into the cell.
